# EUROPEAN PATENT APPLICATION

(11) **EP 4 843 393 A2**
(43) Date of publication of application: **25.11.2026**
(21) Application number: 26178526.5
(22) Date of filing: 13.05.2026
(51) Int. Cl.: A23G 1/02, A23G 1/00, C12N 1/165, C12N 1/205, C12R 1/02, C12R 1/25, C12R 1/84

(54) **A PROCESS FOR COCOA BEAN FERMENTATION**

(30) Priority: 23.05.2025 IT 202500011794
(71) Applicant: SOREMARTEC S.A., 2633 Senningerberg (LU)
(72) Inventor: GUGLIELMETTI, Alessandro, I-10023 CHIERI (Torino) (IT); BORTOLINI, Cristian, I-10024 MONCALIERI (Torino) (IT); GHISOLFI, Rebecca, I-26022 CASTELVERDE (Cremona) (IT); BANDINI, Francesca, I-29122 PIACENZA (IT); PUGLISI, Edoardo, I-29122 PIACENZA (IT); MORELLI, Lorenzo, I-29122 PIACENZA (IT)
(74) Representative: Comoglio, Elena

(57) **Abstract**

The invention relates to a process of fermenting cocoa material (cocoa beans and pulp) based on the inoculation of a freeze-dried starter culture (yeast, lactic acid and acetic acid bacteria) using cocoa pulp as the substrate and on a number of subsequent back-slopping steps. The invention also relates to microorganism strains which were identified as being particularly suitable for use in the process of the invention, as well as to the use of fermented cocoa beans obtainable by the process of the invention for the production of cocoa products and chocolate products.

## Description

The present invention relates to a process of fermenting cocoa material (cocoa beans and pulp) based on the inoculation of a starter culture (yeast, lactic acid and acetic acid bacteria) in cocoa pulp and on a back-slopping technique.

### Background of the invention

The fruit of the *Theobroma cacao* tree, known as the cocoa pod, is large and oval or elongated in shape. Its dimensions vary between 15 and 30 centimeters in length and 7 and 10 centimeters in width. The exterior is a thick, leathery rind. Inside, the pod contains a white, mucilaginous pulp that surrounds the cocoa beans. These beans, arranged in rows within the pulp, are the raw material for the production of chocolate products.

The journey from cocoa bean to chocolate begins with the harvesting of ripe cocoa pods from the *Theobroma cacao* tree. These pods, containing the cocoa beans surrounded by the pulp, are then opened, and the beans are extracted. The crucial step that follows is fermentation, a process that significantly influences the final flavor profile of the chocolate. Traditionally, the beans, along with their pulp, are piled into heaps or placed in boxes, sometimes covered with banana leaves, and left to ferment for several days. During this period, naturally occurring yeasts and bacteria initiate a complex sequence of biochemical reactions. The pulpy mass heats up, reaching temperatures that can exceed 50°C, and the sugars in the pulp are converted into acids, primarily acetic acid. This acidification process, along with enzymatic activities, leads to the breakdown of the bean's cell walls, causing the beans to "die" and initiate the chemical changes necessary for flavor development. Specifically, the bitter-tasting compounds, such as polyphenols, are broken down, and flavor precursors are formed. Following fermentation, the beans undergo drying, traditionally done in the sun, to reduce their moisture content and prevent mold growth. Alternatively, artificial drying in a current of hot-air is performed. Once dried, the beans are shipped to chocolate manufacturers. The subsequent stages involve roasting, which further develops the flavor; winnowing, to remove the shells; and grinding, to create cocoa liquor, a thick, dark paste. This liquor can then be processed into cocoa butter and cocoa solids, the essential ingredients for chocolate. The final steps include mixing, conching (a process that refines the texture and flavor), and tempering (controlled heating and cooling to stabilize the chocolate), ultimately leading to the finished chocolate product.

Fermentation is thus an essential step to mitigate the bitter and astringent taste of fresh cocoa beans, and to develop the characteristic flavor and delicate taste. It plays a significant role in determining the different quality and taste of chocolate and of its by-products and the economic value of cocoa beans (Cempaka et al., 2014).

Cocoa yields are significantly challenged by factors including aging plantations, susceptibility to diseases, and the adverse effects of climate change. While advancements have been achieved in developing hybrid cocoa varieties with enhanced disease resistance, the application of microbial starter cultures is also being investigated as a strategy to meet the growing demand for improved cocoa quality and flavor (Diaz-Muñoz & De Vuyst, 2022).

The *Theobroma* genus encompasses 22 species, with *T. cacao* being the predominantly cultivated species. Forastero, Criollo, and Trinitario (a hybrid of Forastero and Criollo) constitute the three well-defined varietal groups, encompassing various types and cultivars. The Forastero variety represents the most significant commercial or bulk cocoa type and is the most extensively cultivated, particularly in West Africa and Brazil. Forastero trees are characterized by their hardiness and vigor, yielding beans with a robust flavor profile. The pod's hull is relatively smooth and bulbous, contrasting with the furrowed and pointed hull of the Criollo variety. The Forastero pod's hull is also more ligneous, necessitating greater force for opening. Pod color ranges from purple to yellow, and the beans are dark purple and relatively flattened compared to Criollo beans. Forastero beans exhibit lower levels of bitterness, astringency, and acidity than Criollo or Trinitario beans. Criollo trees produce pods with a softer exocarp, containing 20-30 ivory or pale purple beans, and are generally recognized for their superior flavor profile relative to other cacao varieties. These trees display limited disease resistance, rendering them more challenging to cultivate. Criollo, in conjunction with Trinitario, serves as a primary source for "flavor beans," employed to enhance the organoleptic properties of chocolate. Criollo beans are reported to contain elevated levels of pyrazines and exhibit a low pH, factors that significantly influence the resultant flavor profile.

Plant populations and genotypes (cultivars) determine harvest yields and disease resistance, while the interactions between cultivars and the environmental conditions of cultivation significantly influence the composition, flavor, and quality of the fermented dried beans (De Vuyst & Weckx, 2016). Furthermore, flavor variations are contingent upon the conditions governing the fermentation, drying, and roasting processes. A pre-drying step prior to fermentation may enhance the sensory quality, thereby accentuating the fine flavor attributes (Jaimez et al., 2022).

The time elapsed between harvesting and pod opening may significantly impact fermentation. Storage periods exceeding seven days are usually discouraged due to the heightened risk of fungal proliferation at the end of fermentation, potentially leading to mycotoxin formation. Mycotoxins exhibit stability during storage and demonstrate resistance to both chemical and physical treatments; therefore, the most effective strategy for mitigating food contamination is the prevention of their formation. Both ochratoxin A (OTA) and aflatoxins have been detected in cocoa and chocolate products (Copetti et al., 2014). Notably, cocoa bean fermentation has the potential to inhibit undesirable ochratoxigenic fungal species.

Pulp pre-conditioning may modify the properties of the pulp, the substrate for microbial growth, prior to the initiation of spontaneous fermentation, thereby influencing the development and metabolic activity of the microbial consortium involved. The pulp presents a viscous, sweet matrix with a low pH, ranging from 3.0 to 4.0, and comprises approximately 85% water, 10-15% sugars (glucose, fructose, and sucrose, with concentrations varying according to pod age and maturity), 2-3% pentoses, 1-3% citric acid, and 1.5% other components, including pectin, proteins, amino acids, vitamins (predominantly Vitamin C), and minerals (primarily potassium). The removal of pulp fractions or the reduction of fermentable sugar content has been demonstrated to attenuate acid production during fermentation, yielding a positive impact on flavor quality by producing less acidic beans and shortening the fermentation process. Pre-conditioning can be implemented through three primary methodologies: I) pod storage, II) depulping (mechanical or enzymatic), and III) bean spreading. This pre-conditioning step exhibits a significantly beneficial effect on the chemical composition of cocoa beans and the subsequent development of chocolate flavor (Kongor et al., 2016).

According to the conventional procedures, fermentation may occur in heaps, trays, wooden boxes (the predominant method), plastic tanks, or baskets, depending on the cocoa production region, and typically spans 2-10 days, primarily influenced by bean variety and local agricultural practices. Upon completion of fermentation, the beans are either sun-dried or artificially dried to reduce moisture content from approximately 60% to 7%. Drying is a critical process requiring precise control to prevent the development of off-flavors. These processes, from harvesting to drying, are customarily executed on farms located within the producing zones of the "Cacao Belt," prior to exportation to chocolate-producing countries, predominantly situated in Europe and North America.

The spontaneous fermentation of cocoa beans usually involves a consortium of three primary microbial groups: yeasts, lactic acid bacteria (LAB), and acetic acid bacteria (AAB), which synergistically contribute to the production of well-fermented cocoa beans. Their metabolic activities are exothermic reactions, leading to an elevation in temperature of the cocoa pulp-bean mass from 25-30 °C to 45-50 °C, ultimately resulting in a reduction in microbial populations.

To evaluate the degree of fermentation and assess the sanitary quality of the beans, the cut test remains the simplest and most widely employed method. This test enables the identification of beans exhibiting visible mold, unfermented (slaty) characteristics, infestation, or germination. The cut test evaluates the degradation of anthocyanins present in the cocoa beans; gray and violet coloration typically indicates inadequate fermentation, while brown beans signify a satisfactory fermentation process.

Controlled fermentation utilizing appropriate starter cultures is a known technique, aimed at accelerating the fermentation process, augmenting production to meet the escalating demand for cocoa (considering the impact of climate change on cocoa plantations), and directing fermentation towards standardized and enhanced flavor profiles in the final products (Gutiérrez-Ríos et al., 2022).

Efforts to develop efficient starter cultures for cocoa fermentation commenced in the preceding century, driven by the objective of producing high-quality chocolates with consistent characteristics and enhanced sensory attributes. Subsequently, various yeast strains have been proposed as potential candidates, acknowledging the critical role of this microbial group in the fermentation process. A comprehensive review by Figueroa-Hernández et al., 2019 consolidated forty-seven studies published between 2008 and 2018, detailing the cocobiota from 13 distinct countries, with the aim of facilitating the design of starter cultures to standardize cocoa bean fermentation.

Yeast starter fermentation trials have been conducted worldwide in equatorial cocoa-producing regions, examining various cocoa varieties (Forastero, Trinitario, and hybrids) and fermentation methods. Typically, an exhaustive screening of candidate yeast starter cultures is performed to identify optimal strains for controlling fermentation and enhancing specific characteristics, such as pectinolysis, ethanol production, citrate assimilation, and flavor development.

The yeast species most used as starters for cocoa fermentation are *Saccharomyces cerevisiae, Pichia kluyveri, Pichia kudriavzevii, Kluyveromyces marxianus, and Torulaspora delbrueckii.* The use of *Saccharomyces* species is linked to their ability to assimilate citric acid and to reduce sugars, killer toxins and volatile organic compounds (VOCs), and pectinolytic activity. While *non-Saccharomyces* yeasts have relevant pectinolytic activity and are associated with increased aroma complexity in wine (Gutiérrez-Ríos et al., 2022).

Due to the spontaneous nature of cocoa fermentation, the resulting fermented and dried product is often contaminated, of low quality, and prone to production waste. As the commercial value depends on the quality of the fermented beans themselves, it is important to understand how to guide this fermentation. Additionally, standardizing the fermented and dried product would improve subsequent processing, since the final product is highly variable, and chocolate companies need to mix various types of cocoa to achieve their standards.

To address these issues, there is a need to control the cocoa bean fermentation process, in order to improve the quantity and quality of fermented and dried beans, decrease waste and process variability, resulting in a standardized cocoa-based product.

WO 2009/138419 discloses a method for processing unfermented cocoa beans wherein the cocoa beans are depulped and wherein separated pulp and depulped cocoa beans are separately treated. In particular, the method comprises a fermentation of pulp and at least one treatment of depulped cocoa beans under acidic conditions, followed by preparing a mixture of fermented pulp and acid-treated depulped cocoa beans and the further processing of such mixture.

However, existing methods, such as those described in WO2009138419 A2, may not fully exploit the potential of repeated back-slopping to enhance fermentation consistency and quality. This limitation can result in variations in the final product quality.

The present invention further aims to implement a fermentation process utilizing selected microorganism strains, alone or in mixtures, chosen for their beneficial characteristics. This controlled fermentation environment for cocoa beans will standardize results and minimize waste and variability associated with natural fermentation.

### Summary of the invention

These and other objects are achieved by the innovative cocoa bean fermentation process as defined in claim 1, which is essentially based on the inoculation of a starter culture using cocoa pulp as the substrate and on a number of subsequent back-slopping steps.

The innovation also concerns the products that arise from implementing this method.

The cocoa bean fermentation process of the invention advantageously achieves consistent and high-quality cocoa bean fermentation. By incorporating a series of back-slopping steps, the process stabilizes the microbial population and fermentation conditions, leading to a more uniform fermented cocoa bean product.

Another aspect of the present invention is a process of preparing a cocoa product or a chocolate product comprising the steps of (i) fermenting cocoa beans by the process of the invention thereby obtaining a cocoa bean fermentation product, and (ii) using the cocoa bean fermentation product in the preparation of a cocoa product or a chocolate product.

By way of a non-limiting example, a cocoa product is cocoa nibs, cocoa powder, cocoa extract, cocoa liquor, cocoa mass, cocoa butter, or cocoa cake.

By way a non-limiting example, a chocolate product is a chocolate bar, chocolates, truffles, pralines, raw chocolate, stone-ground chocolate, aerated chocolate, chocolate chips, chocolate nuggets, or chocolate spreads.

A further aspect of the present invention are the yeast, lactic acid bacteria (LAB) and acetic acid bacteria (AAB) strains isolated by the present inventors as the best-performing strains in the process of the invention, which have been successfully cultivated on cocoa bean pulp using the back-slopping technique as defined above.

The strain selection process developed by the inventors included the isolation of microorganisms from cocoa fermentations in Cameroon and Ivory Coast and phenotypic screening to select high-performing strains resistant to various stresses, including temperature, acidity, and ethanol. Further enzymatic analyses were performed. The selection process utilized a ranking system, where strains were scored based on their resistance to each stress treatment. Each isolate was assigned a score from 1 to 5, corresponding to its OD600 measurement, with 1 representing the lowest OD600 and 5 the highest for each treatment. Enzymatic activity results were similarly ranked, assigning quintile scores to the difference between results and the blank.

A further aspect lyophilized starter culture comprising a preferred yeast, lactic acid bacteria (LAB) or acetic acid bacteria (AAB) strain or a combination thereof. The lyophilized starter culture may comprise cryoprotecting agents as needed.

### Detailed description of the invention

The process for cocoa bean fermentation of the invention and preferred features thereof are further described by means of the following embodiments and with reference to the appended drawings.

The appended claims for an integral part of the present description.

The term "cocoa" as used in the present description is intended to include any variety or hybrid of *Theobroma cacao.*

The term "cocoa beans" as used in the present description refers to cocoa beans or cocoa seeds as such, as well as parts thereof and includes cocoa nibs.

The term "cocoa pulp" as used in the present description refers to the mucilaginous plant material in which cocoa beans are embedded inside the cocoa pods.

The term "de-pulping" as used in the present description refers to the process of removing the pulp that surrounds the cocoa beans within the harvested cocoa pod.

The term "back-slopping" as used in the present description refers to using a portion of a previously prepared fermented cocoa pulp as the fermentation inoculum for freshly prepared cocoa pulp, so as to obtain further fermented cocoa pulp. In the process of the invention, said inoculation and fermentation operations are repeated multiple times (n-times).

The process for cocoa bean fermentation of the invention includes step a) of de-pulping, in which the pulp that surrounds the cocoa beans is separated from the cocoa beans, so as to obtain cocoa pulp.

In a preferred embodiment, cocoa pulp is obtained by de-pulping substantially unfermented cocoa beans. Preferably, cocoa pulp is obtained from cocoa beans de-pulped quickly after pod harvest. This minimizes microbial processes that typically begin during storage or fermentation. This can be achieved by removing cocoa beans from healthy pods quickly, preferably within 5 days, and no more than 10 days after harvest. The cocoa beans are then immediately de-pulped. Several known methods exist for de-pulping cocoa beans, including mechanical means, enzymatic de-pulping and washing.

Step b) of the cocoa bean fermentation process of the invention comprises pasteurizing the cocoa pulp obtained from step a).

Cocoa pulp pasteurization is a process designed to decrease the microbial load within cocoa pulp, thereby extending its shelf life and ensuring safety for consumption or further processing. Given cocoa pulp's rich sugar and nutrient content, it is highly susceptible to microbial growth, which can lead to spoilage. Pasteurization addresses this by eliminating or significantly reducing harmful microorganisms, including bacteria, yeasts, and molds, that contribute to deterioration and pose health risks. Additionally, it aims to deactivate enzymes that could degrade the pulp's quality.

In step b) of the process of the invention, cocoa pulp pasteurization is performed by any known method. For example, it is pasteurized by heat treatment, which involves heating the cocoa pulp to a specific temperature for a defined time period. This can be achieved through techniques like high-temperature short-time (HTST) pasteurization, which involves heating the pulp to a high temperature for a short duration, or low-temperature long-time (LTLT) pasteurization, which uses a lower temperature for a longer period. Alternative technologies for cocoa pulp pasteurization are also known, such as high-pressure processing.

Step c) of the cocoa bean fermentation process of the invention comprises inoculating the pasteurized cocoa pulp with one or more freeze-dried fermentation starter cultures, wherein each of the one or more freeze-dried fermentation starter cultures comprises one or more microorganism strains selected from the group consisting of yeast strains, lactic acid bacteria (LAB) strains and acetic acid bacteria (AAB) strains.

Freeze-drying is a widely used method for long-term storage of biological materials. The process involves freezing the sample, applying a high vacuum, warming under vacuum to sublimate water, removing excess moisture during drying, and sealing the sample to prevent rehydration. Protective solutes, such as cryoprotectants (FDPA - Freez-Drying Protective Agent-: 20% Skim milk power (Oxoid, LP0033B), 10% D(+)-Sucrose (Carlo Erba, 477187), are essential to preserve cell membrane integrity and viability. The effectiveness of each protective agent varies according to the strain and should be evaluated individually.

Freezing temperature is also a critical factor in the freeze-drying process. Slow freezing allows water to exit the cells by osmosis, forming extracellular ice crystals, while rapid freezing can lead to intracellular ice formation, damaging the cells. The optimal freezing rate depends on the microorganism genus.

In a preferred embodiment, the inoculation of step c) is carried out by three subsequent freeze-dried starter culture inoculations, wherein the first inoculation is made with a freeze-dried starter culture comprising one or more yeast strains, the second inoculation is made with a freeze-dried starter culture comprising one or more LAB strains, and the third inoculation is made with a freeze-dried starter culture comprising one or more AAB strains.

By way of a non-limiting example, the pasteurized cocoa pulp is inoculated with a yeast starter culture at time zero (T0h), then after approximately 24 hours from time zero (T24h) it is inoculated with the LAB starter culture, and then after approximately 48 hours from time zero (T48h) it is inoculated with the AAB starter culture.

The aforementioned preferred embodiment results from the analysis of the features and behavior of yeasts, lactic acid bacteria and acetic acid bacteria in the fermentation of cocoa pulp.

Yeasts are eukaryotic microorganisms, resistant to antibiotics, sulfa-drugs, and other antibacterial agents. This resistance is genetic and cannot be modified or transmitted to other microorganisms. During the cocoa fermentation, yeasts are active for around 48h, with their activity peaking at 24-hours. By this point, they have altered their environment significantly and are crucial in the process of pulp liquefaction, which then drains away (Ordoñez-Araque et al., 2020). When the pulp becomes less viscous, it allows air to enter, leading to the conversion of the mucilage's simple sugars into ethanol. This degradation of pectin alters the bean's texture and removes citric acid. The yeasts typically responsible for metabolizing citric acid are *Candida* spp. and *Pichia* spp., which create an alkaline pH. The alkaline environment, along with the presence of alcohol and oxygen, inhibits the yeasts and supports the growth of lactic acid bacteria (Ordoñez-Araque et al., 2020).

Lactic acid bacteria (LAB) are Gram-positive, non-motile microorganisms that lack catalase and oxidase enzymes and do not form spores. As the pulp drains and more oxygen is introduced, conditions become ideal for LAB growth. LABs are active during the first 72 hours of fermentation, reaching their peak activity at 36 hours, with the most significant growth occurring between 16 and 48 hours, either in anaerobic or microaerophilic conditions. Their primary metabolite, lactic acid, diffuses into the seeds and remains there even after drying and roasting, influencing the acidity of the beans. This process is important to activate the endogenous enzymes that contribute to the characteristic aroma and flavor of chocolate.

The over production of lactic acid is not desirable, as it can lead to an excessive acidity in cocoa seeds (Viesser et al., 2021). The main mechanism of lactic acid production is attributed especially to heterofermentative species such as *L. plantarum,* and less to obligate heterofermentative species like *L. fermentum,* that convert the major part of fructose to mannitol instead of lactic acid (Ho et al., 2015).

Acetic acid bacteria are Gram-negative and they are strict aerobics, do not form spores and have ellipsoidal or bacillus shapes. Following the liquefaction of the pulp and the introduction of oxygen into the environment, acetic acid bacteria (AAB) can oxidize the ethanol produced earlier into acetic acid. Some AAB begin to appear around 24 hours and reach their maximum growth at 88 hours. By 120 hours, they are no longer detectable (Ordoñez-Araque et al., 2020). They cause the death of the seed embryo.

According to the process of the invention, the freeze-dried fermentation starter culture, or each of the freeze-dried fermentation starter cultures, is/are inoculated in the pasteurized cocoa pulp either with or without prior rehydration.

In a preferred embodiment, the microorganism concentration in the freeze-dried fermentation starter culture sample(s) used for inoculation varies within the range of from 10⁵ to 10¹⁰ CFU/grams. Such sample(s) are inoculated in the pasteurized cocoa pulp in a preferred amount of about 1% (w/w).

The yeast strains which are preferred for use as a freeze-dried fermentation starter culture in the process of the invention are from the species *Pichia kudriavzevii.*

The LAB strains which are preferred for use as a freeze-dried fermentation starter culture in the process of the invention are from the species *Lactiplantibacillus plantarum.*

The AAB strains which are preferred for use as a freeze-dried fermentation starter culture in the process of the invention are from the species *Acetobacter malorum.*

Within the aforementioned microorganism species, the following strains are particularly preferred, which have been isolated and identified by the present inventors as disclosed in more detail in the following experimental section:
*Lactobacillus plantarum,* accession number DSM 35385, deposited at DSMZ on March 6, 2025;
*Acetobacter malorum,* accession number DSM 35383 deposited on March 6, 2025;
*Pichia kudriavzevii,* accession number DSM 35384, deposited on March 6, 2025.

Such preferred strains were deposited at DSMZ under the Budapest Treaty.

Step d) of the cocoa bean fermentation process of the invention comprises incubating the inoculated pasteurized cocoa pulp for a time and temperature sufficient to obtain a mother culture of fermented pulp.

Incubation time and temperature, as well as other parameters relevant to fermentation such as pH, humidity and oxygen availability, are known to depend on the microorganism and the environmental conditions. A person skilled in cocoa pulp fermentation is able to identify the most appropriate values for these parameters according to the specific circumstances in which fermentation takes place. By way a non-limiting example, an appropriate time for incubation is of approximately 24 hours to 50 hours, preferably of approximately 48 hours, at a temperature of between 25°C to 40°C.

The mother culture of fermented pulp obtained in step d) is suitable for being stored for an extended period of time in a refrigerator (i.e. at about 4°C), whereafter it may be used as the starting inoculum for the fermentation of fresh pasteurized cocoa pulp to obtain new mother culture stocks. The possibility of storing the mother culture in non-extreme cooling conditions is a clear advantage, considering that the countries where cocoa is harvested and where the fermentation of cocoa beans takes place are usually hot countries where maintaining a very strict cold chain could be a problem.

The subsequent steps e) and f) of the cocoa bean fermentation process of the invention constitute the back-slopping steps.

In step e), an aliquot of the mother culture of fermented pulp obtained in the preceding step d) is taken and used to inoculate pasteurized fresh cocoa pulp, which is then incubated for a time and at a temperature sufficient to obtain a first fermented pulp.

The term "pasteurized fresh cocoa pulp" as used in the present description refers to substantially unfermented cocoa pulp, which has been pasteurized as described above.

As described for step d), incubation time and temperature, as well as the other fermentation parameters, depend on the specific circumstances and conditions under which fermentation takes place, and their appropriate selection is within the capabilities of the skilled person. By way a non-limiting example, an appropriate time for incubation is of approximately 24 hours to 50 hours, preferably of approximately 48 hours, at a temperature of between 25°C to 40°C.

Step f) of the process of the invention consists in the repetition of step e) for a number n of times, each time by inoculating a freshly prepared pasteurized cocoa pulp with an aliquot of the fermented pulp obtained from the corresponding preceding step to obtain a second, third and up to nth fermented pulp, until the cell count of the inoculated microorganism in the last obtained fermented pulp remains substantially stable.

Viable cell counting methods, such as viability staining, fluorescence microscopy and RNA-targeted qPCR, are known to the skilled person.

Following each of the n repetitions of step (e), the resulting fermented pulp may optionally be refrigerated, as previously discussed with reference to the mother culture. This approach is appropriate for example when cocoa bean fermentations are not performed daily.

Step g) of the process of the invention comprises adding a first aliquot of the first fermented pulp and/or of any of the fermented pulps subsequently obtained in step f), to cocoa beans for in-field fermentation and fermenting to obtain a cocoa bean fermentation product.

According to the process of the invention, said cocoa beans used in step g) are fermented or unfermented beans. Optionally, before performing step g), the cocoa beans are dried, either artificially in a current of hot-air or by sun-drying.

In one embodiment, the fermented pulp is diluted, preferably with water, prior to or during addition to cocoa beans for in-field fermentation. Alternatively, undiluted pulp is used for the in-field fermentation of cocoa beans. A suitable ratio of fermented pulp (g dry matter) to cocoa beans (g dry matter) for in-field fermentation is of 1:1 to 1:100.

The in-field fermentation of the cocoa beans is carried out according to fermentation methods known per se. Typically, freshly harvested cocoa pods are opened, and the beans, still encased in their mucilaginous pulp, are extracted. The fermented pulp is typically spread throughout the fresh bean mass, ensuring even distribution of the microbial community. This can be done e.g. by layering the fermented pulp amongst the beans, or by mixing it in as the beans are piled or placed into fermentation containers like boxes or heaps optionally covered with jute sacs and/or black plastic films. The goal is to maximize contact between the established microbial population in the fermented pulp and the cocoa beans to be fermented. The duration of cocoa bean fermentation may vary according to a number of factors, including the bean variety, the fermentation method and the environmental condition. A typical duration is between 2 to 10 days, more typically between 5 and 7 days. During this period of time it is usual for the cocoa beans to be stirred approximately every two days.

In an embodiment of the process of the invention, a second aliquot or the remaining part of said first fermented pulp is collected, optionally stored at refrigerator temperature and, after the optional period of storage, it is used as the starting inoculum for in-field cocoa bean fermentation.

In a further embodiment of the process of the invention, a second aliquot of at least one or each of the fermented pulps created from step f), after an optional period of storage at refrigerator temperature, is used as inoculum for in-field cocoa bean fermentation.

A further aspect of the invention is the use of the bean fermentation product obtainable by the process of the invention for the preparation of cocoa products or chocolate products. Non-limiting examples of cocoa products are cocoa powder, cocoa extract, cocoa liquor, cocoa mass, cocoa butter and cocoa cake. Methods for preparing cocoa products and chocolate products from fermented cocoa beans are known to the skilled person, as outlined in the background section of the present description. The use of cocoa beans obtainable by the process of the invention as the starting materials advantageously results in cocoa products and chocolate products having a controllable and well-defined composition.

Still another aspect of the invention are the microorganism strains which have been identified by the inventors as being particularly suitable for use as a starter culture in the cocoa bean fermentation process of the invention.

Such strains are:
*Lactobacillus plantarum, accession number DSM 35385, deposited at DSMZ on March 6, 2025;*
*Acetobacter malorum, accession number DSM 35383 deposited on March 6, 2025;*
*Pichia kudriavzevii, accession number DSM 35384, deposited on March 6, 2025.*

The following examples are provided by way of illustration only and are not intended to limit the scope of the invention as defined by the appended claims.

### Examples

### 1. Phenotypic tests

### 1.1 Phenotypic screening: protocols for yeasts, LABs and AABs

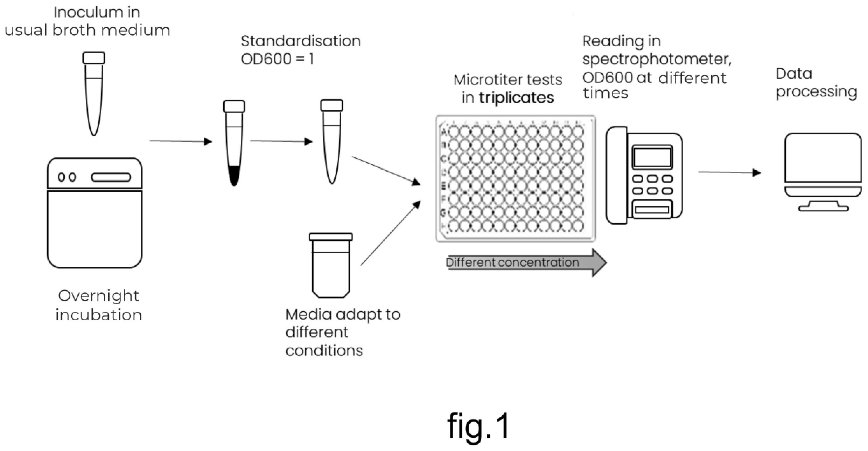

A schematic overview of the phenotypic screening process for yeasts, LABs and AABs which was used to identify the strains that have a greater capacity to tolerate the applied stress conditions is provided in Figure 1.

Isolates were cultured in their usual growth medium (MEB - Malt Extract Broth, CM0057B, Oxoid- for yeast strains, MRS - DeMan, Rogosa y Sharpe CM0359B, Oxoid- broth for LAB strains and AAM -1% d-glucose, 0.5% ethanol, 0.3% acetic acid, 1.5% peptone, 0.8% yeast extract, wt/vol - broth for AAB strains) and incubated overnight. To standardize the inoculum for all the phenotypic tests, cell density was adjusted and made equal either by diluting the culture with clean sterile broth or, where necessary, by concentrating the culture, pelleting the cells, and discarding some media. The cell density was properly determined using a spectrophotometer, measuring the absorbance level at a wavelength of 600 nm. Once the optical density at 600 nm (OD₆₀₀) was adjusted to the value of 1.00, it was used to inoculate the microtiter wells. This allowed the inoculation to reach approximately an OD₆₀₀ = 0.2 corresponding to a starting cell number of ~10⁶ cells/mL (Arroyo-lópez et al., 2010). Data obtained from the OD₆₀₀ absorbance readings after incubation were collected from the multi-plate reader software (Gen5^{™}) and initially elaborated with Excel. Statistical analysis was performed using the CoStat software, while averages between the triplicates and standard deviation were calculated and plotted to better appreciate the differences between the strains' OD₆₀₀. The Tukey's (P < 0.05) test was performed on replicates to evaluate the statistical differences. All the tests were performed in triplicates.

The stressors were chosen to simulate the fermentation conditions, in accordance with the data reported in the scientific literature. Strains with greater tolerance towards adverse conditions have a higher probability of survival in the fermentation process.

The phenotypic screening of yeast strains was designed to test the tolerance of each strain towards an increasing concentration/gradient of ethanol (5, 10, 15 and 20%), acidity (pH 5, 4, 3.5, 3 and 2.5, lowered with lactic acid), and temperature (37, 45, 50 and 55 °C).

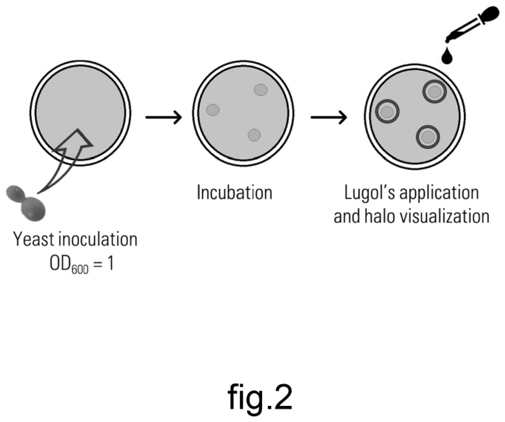

In addition, yeast pectinolytic activity was determined by inoculation in triplicate on mineral medium agar plates with pectin (2.0 g/L). The basal minimal medium composition was: MnSO₄ 0.05 g/l, KH₂PO₄ 0.2 g/l, (NH₄)₂SO₄ 1 g/l, CaCl₂ 0.05 g/l, MgSO₄7*H₂O 0.8 g/l, yeast extract 1.0 g/l, citric pectin 2.0 g/l. (Schwan et al., 1997). A schematic overview of the protocol employed for the determination of yeast pectinolytic activity is provided in Figure 2. Enzyme activity was shown by the development of a clear color change (yellow) around the colonies after flooding the plate with Lugol's (iodide-iodine solution).

The Pectin Degradation Index (PDI) % was determined by measuring the diameter of the yeast colonies, and the pectin degradation zones were measured using a ruler after five days of incubation. The area degraded by enzymatic activity after incubation at 25°C for 7 days showed a clear yellow zone around the colonies upon flooding with 50 mM potassium iodide-iodine solution.

The tolerance capacity of each LAB strain towards an increasing concentration/gradient of ethanol (5, 10, 15 and 20 %), acidity (pH 5, 4, 3.5, and 3, lowered with acetic acid), and temperature (45, 50 and 55 °C) was tested. In addition to stress tolerance, LAB β-galactosidase activity was determined (Watson & Chiu, 2016). β-galactosidase is a key enzyme involved in the flavor precursors formation from the degradation of polyphenols (Castro-Alayo et al., 2019). Indeed, the pulp is the base substrate for microorganisms during fermentation and linalool is one of its constituents, which is transferred to the beans during fermentation. Linalool is a terpene and a plant aroma compound that contains a glycoside bond and contributes to the flowery and tea-like flavor. Glycosidase enzymes, such as β-galactosidase, play an important role in the release of these compounds. Other key enzymes that play role in the flavor precursor and polyphenol reduction are aspartic endoprotease, carboxypeptidase, polyphenol oxidase, invertase, α-arabinosidase and α-mannosidase.

The tolerance capacity of AAB strain towards an increasing concentration/gradient of acidity (pH 5, 4, 3.5, 3, and 2.5, lowered with citric acid) and temperature (45, 50 and *55* °C) was tested. In addition to stress tolerances, mannitol and lactic acid oxidation and acetic acid over-oxidation were determined on AAB isolates. AABs involved in acetic acid fermentation exhibit two different acetic acid resistance phases: one is the ethanol oxidation phase, which is characterized by oxidation of ethanol to acetic acid, where acetic acid resistance occurs without acetate assimilation, and the second phase is the overoxidation phase, which is characterized by oxidation of acetic acid to water and carbon dioxide, where the cells overcome acetic acid by its assimilation. The overoxidation occurs in *Acetobacter* and *Gluconacetobacter,* but not in *Gluconobacter,* which exhibits a relatively weak acetic acid resistance. *Gluconobacter* genus can oxidize a broad range of sugars, sugar alcohols, and sugar acids, and accumulate a large amount of the corresponding oxidized products in culture medium.

Data collected were elaborated following a ranking system that allows the selection of the most performing strains summing, in the end, the score awarded for the capability to resist each stress (temperature, ethanol, acid conditions). The same method was also performed on enzymatic activity or oxidation results.

Each isolate was assigned a number from 1 to 5, corresponding to one of five quintiles, determined by dividing the range between the highest and lowest OD₆₀₀ measurements into equal parts. For each treatment, a score of 1 was assigned to the lowest OD₆₀₀ value and a score of 5 to the highest.

The reading times used to develop the ranking system were selected to best represent full-scale applications (e.g., 24-48 hours for yeast and LAB, 48-72 for AAB). Likewise, stress conditions were chosen to closely mimic real fermentation conditions.

### Results

The best performing yeast selected (DSM 35384) belongs to the species *Pichia kudriavzevii,* scoring 5 for temperature, pH resistance and PDI%, 4 for resistance to 5-10% of ethanol. Pectinolytic activity is important at the beginning of the fermentation, as it facilitates air permeability in the fermenting cocoa beans mass, allowing oxygen to penetrate and enabling the development of LAB, which further continue the fermentation process. As reported (Viesser et al., 2021), only some *P. kudriavzevii* strains produce some pectinolytic enzymes. *P. kudriavzevii* produces ovoid or elongated ellipsoidal cells that resemble long-grain rice.

The strain was isolated, grown and re-assessed on plate of MEA (Malt Extract Agar, CM0001B, Oxoid) or resuspended on MEB (Malt Extract Broth, CM0059B, Oxoid) medium. It was grown in aerobic conditions, at 30°C for 24 hours. Conditions for long term storage are MEB+20% glycerol at -20, -40 or -196°C.

Table 1 below shows the yeast strains' final ranking for phenotypic tests and pectinolytic activity (PDI%). Each OD₆₀₀ value was calculated as an average between 24- and 48-h. The intensity of the grey color of each cell's background depends on the corresponding score value. Next to each test, the scores are reported according to the previously described method. The last column in the table reports the total score, which is the sum of the scores for each test, including the PDI%. The 3 winning strains are highlighted in bold and with a darker grey background.

**Table 1**

| **ID** | **37-45°C** | score | **5-10% EtOH** | score | **pH3** | score | **PDI%** | score | **Total score** |
|---|---|---|---|---|---|---|---|---|---|
| ***S. cerevisiae*** | 0.431 | 1 | 1.429 | 5 | 0.923 | 4 | 250 | 3 | 13 |
| ***S. cerevisiae*** | 0.576 | 1 | 0.899 | 3 | 1.094 | 5 | 225 | 2 | 11 |
| ***S. cerevisiae*** | 0.514 | 1 | 0.545 | 1 | 0.824 | 3 | 200 | 1 | 6 |
| ***P. mandshurica*** | 0.895 | 3 | 0.670 | 2 | 0.502 | 1 | 253.33 | 4 | 10 |
| ***P. mandshurica*** | 1.137 | 4 | 0.616 | 1 | 0.433 | 1 | 0 | 0 | 6 |
| ***P. kudriazevii*** | 1.362 | *5* | 1.037 | 4 | 1.025 | 5 | 288.23 | 5 | **19** |
| ***P. kudriazevii*** | 1.001 | 4 | 1.028 | 4 | 1.042 | 5 | 282.35 | 5 | **18** |
| ***P. brunensis*** | 0.702 | 2 | 0.545 | 1 | 1.128 | 5 | 200 | 1 | 9 |
| ***W. anomalus*** | 0.647 | 2 | 0.990 | 3 | 1.098 | 5 | 0 | 0 | 10 |
| ***S. cerevisiae*** | 0.490 | 1 | 0.950 | 3 | 0.556 | 1 | 0 | 0 | 5 |
| ***P. kudriazevii*** | 1.085 | 4 | 0.753 | 2 | 1.102 | 5 | 287.5 | 5 | **16** |
| ***P. mandshurica*** | 0.473 | 1 | 0.425 | 1 | 0.591 | 2 | 0 | 0 | 4 |

The best performing LAB selected (DSM 35385) belongs to the species *Lactiplantibacillus plantarum,* scoring 5 for temperature and pH, 4 for ethanol resistance. For our purposes, enzymatic activity is not as significant as stress resistance, which is why this strain was selected, despite a low score in β-galactosidase. The results of this test are not reported in Table 2 below.

The strain was isolated, grown and re-assessed on plate of MRS (De Man-Rogosa-Sharpe, CM0359B, Oxoid) agar and broth medium. It was grown in anaerobic conditions, at 37°C for 24 hours. Conditions for long term storage are MRS broth+20% glycerol at -20, -40 or - 196°C.

Table 2 below shows the LAB strains' final ranking for phenotypic tests. Each OD₆₀₀ value was calculated as an average between 24- and 48-h. The intensity of the grey colour of each cell's background depends on the corresponding score value. Next to each test, the scores are reported according to the previously described method. The last column in the table reports the total score, which is the sum of the scores for each test. The 3 winning strains are highlighted in bold and with a darker grey background.

**Table 2**

| **ID** | **45-55°C** | score | **15-20%EtOH** | score | **3.5 pH** | score | **Total score** |
|---|---|---|---|---|---|---|---|
| ***L. plantarum*** | 1.011 | 3 | 0.361 | 1 | 0.505 | 4 | 8 |
| ***L. plantarum*** | 0.839 | 1 | 0.369 | 1 | 0.542 | 5 | 7 |
| ***L. plantarum*** | 1.050 | 3 | 0.416 | 3 | 0.464 | 2 | 8 |
| ***L. plantarum*** | 0.962 | 2 | 0.337 | 1 | 0.549 | 5 | 8 |
| ***L. plantarum*** | 1.010 | 3 | 0.410 | 3 | 0.404 | 1 | 7 |
| ***L. plantarum*** | 0.930 | 2 | 0.383 | 2 | 0.495 | 3 | 7 |
| ***L. fermentum*** | 1.192 | 5 | 0.428 | 3 | 0.518 | 4 | **12** |
| ***L. fermentum*** | 1.114 | 4 | 0.501 | 5 | 0.413 | 1 | 10 |
| ***L. fermentum*** | 1.166 | 4 | 0.421 | 3 | 0.472 | 3 | 10 |
| ***L. fermentum*** | 1.243 | 5 | 0.432 | 3 | 0.554 | 5 | **13** |
| ***L. plantarum*** | 1.165 | 5 | 0.447 | 4 | 0.560 | 5 | **14** |

The best performing AAB strain selected (DSM 35383) belongs to the species *Acetobacter malorum* and scored totally 18/25.

It was isolated, grown and re-assessed on plate of AAM (1% d-glucose, 0.5% ethanol, 0.3% acetic acid, 1.5% peptone, 0.8% yeast extract, wt/vol) agar (1.5% agar) and broth medium. It was grown in aerobic conditions, at 30°C for 48 hours. Conditions for long term storage are AAM broth+20% glycerol at -20, -40 or -196°C.

Table 3 below shows the AAB strains' final ranking for phenotypic tests and mannitol+lactic acid oxidation and acetic acid over-oxidation tests. Each OD₆₀₀ value was calculated as an average between 48- and 72-h. The intensity of the grey colour of each cell's background depends on the corresponding score value. Next to each test, the scores are reported according to the previously described method. The last column in the table reports the total score, which is the sum of the scores for each test, including the mannitol+lactic acid oxidation and acetic acid over-oxidation tests. The 3 winning strains are highlighted in bold and with a darker grey background.

**Table 3**

| **ID** | **pH 3** | score | **50-55°C** | score | **Lactic acid** | score | **Acetic acid** | score | **Mannitol** | score | **Total score** |
|---|---|---|---|---|---|---|---|---|---|---|---|
| ***A. malorum*** | 0.175 | 4 | 0.142 | 5 | 6.915 | 2 | -4.815 | 4 | 12.798 | 2 | **17** |
| ***A. tropicalis*** | 0.217 | 5 | 0.128 | 4 | 6.590 | 2 | -21.719 | 1 | 33.093 | 4 | **16** |
| ***A. tropicalis*** | 0.056 | 1 | 0.111 | 2 | 6.182 | 2 | -5.169 | 4 | 41.139 | 5 | 14 |
| ***A. pasteurianus*** | 0.146 | 3 | 0.131 | 4 | 2.848 | 1 | -0.352 | 5 | 2.934 | 1 | 14 |
| ***A. pasteurianus*** | 0.211 | 5 | 0.113 | 2 | 6.993 | 2 | -2.350 | 5 | 7.699 | 1 | 15 |
| ***G. oxydans*** | 0.105 | 2 | 0.099 | 1 | 8.614 | 3 | -2.123 | 5 | 21.049 | 3 | 14 |
| ***A. pasteurianus*** | 0.176 | 4 | 0.120 | 3 | 4.636 | 1 | 2.352 | 5 | 11.844 | 2 | 15 |
| ***A. pasteurianus*** | 0.173 | 4 | 0.117 | 3 | 8.295 | 3 | -14.095 | 1 | 9.515 | 1 | 12 |
| ***A. malorum*** | 0.122 | 3 | 0.121 | 3 | 16.176 | *5* | -9.087 | 3 | 29.917 | 4 | **18** |

### 1.2 Genomic analysis for the best-performing bacteria

After strains cultivation and total DNA extraction, genomic DNA was sequenced at Fasteris facilities (Geneve, Switzerland). The library was fragmented at 350 bp size and then sequenced with a MiSeq instrument (Illumina Inc., San Diego, US) operating with V3 chemistry in paired reads 300x2 bp. The raw sequences in FASTQ format obtained after the MiSeq run were firstly assigned to the genomic libraries by identification and trimming of the 6 bp Illumina tag used for its sequencing. The resulting paired-end reads files (2 for each isolate) were submitted to the comprehensive genome analysis (CGA) service at PATRIC.

Whole Genome Sequencing enables the identification of genes related to stress response, bacteriocin biosynthesis, and safety assessment by detecting antibiotic resistance genes and virulence factors. The online database ResFinder (Zankari et al., 2012) was used to identify acquired antimicrobial resistance (AMR), a critical global threat. To assess virulence factors, the VFDB database (Chen et al., 2005) was used. Additionally, Bagel4 was used to identify bacteriocin ORFs in DNA sequences, revealing a strain's potential to produce bacteriocins-antimicrobial peptides effective against a wide range of microbes.

### Results

The genome analysis of the three preferred stains (DSM 35384; DSM 35385; DSM 35383) showed positive characteristics of *Acetobacter malorum* (DSM 35383), which produces bacteriocins that may give an advantage during fermentation; no antibiotic-resistance genes were found according to ResFinder database.

All the experiments described in sections 2, 3 and 4 below were carried out with the best-performing strains: *Lactobacillus plantarum,* accession number DSM 35385, deposited at DSMZ on March 6, 2025; *Acetobacter malorum,* accession number DSM 35383 deposited on March 6, 2025; *Pichia kudriavzevii,* accession number DSM 35384, deposited on March 6, 2025.

### 2. Freeze-dried inoculum shelf life

### 2.1 Freeze-drying

Each strain (DSM 35384; DSM 35385; DSM 35383) was freeze-dried following a specific protocol tailored to its growth conditions, including growth time, temperature, and freezing time. This approach was designed to ensure maximum efficiency for each process.

Each strain was cultured in their best-performing media (MEB, Malt-Extrat Broth - CM0057B, Oxoid- for yeast, MRS broth - CM0359B, Oxoid- for LAB and AAM broth for AAB) at 30°C aerobiosis conditions for yeast and AAB and 37°C in anaerobiosis conditions for LAB strains. When the microorganism cells had grown up to early stationary phase, the cells were collected by centrifugation (5000 rpm for 15 min). The harvested pellet is added in proportion 1:4 (w/w) by FDPA (Freeze-Drying Protective Agent): 20% SPM (Skimmed Milk Powder, LP0031, Oxoid) + 10% sucrose. The suspension was mixed, to allow a well homogenisation, put in plates and frozen at -20°C for 24h. After the overnight freezer storage, plates were put in the lyophiliser. The freeze-dried strains were collected in sterile bags and stored in a dry place.

Before inoculation into the fermenting mass, the freeze-dried strains were carefully rehydrated. The samples were rehydrated by weighting 1 gr of freeze-dried strain and adding 9 ml of 9% NaCl solution at environment temperature. Then, samples were carefully homogenized for 1-2 min with a Vortex mixer and incubated at room temperature for 10 min. To determine the viability of the cells, serial dilutions were spread-plated on different agar medium plates, according to the microorganisms: MEA for yeast, MRS for LAB and AAM for AAB. Plates were incubated for 48h at 30°C in aerobiosis conditions for yeast and AAB and 37°C in anaerobiosis condition for LAB. Plates reading results expressed in colony-forming units per millilitre (CFU/ml).

### 2.2 Shelf-life monitoring

To satisfy field requirements, freeze-drying tests were performed at different temperatures (room, refrigerator, and freezer temperature), with monthly counts to monitor viability and possible contamination. The test was done in triplicates and the reads after 24 and 48 hours.

Table 2 below shows the freeze-dried strains (DSM 35384; DSM 35385; DSM 35383) shelf-life at T0, T1 (1 month of storage), T2 (3 months of storage), T3 (4 months of storage), T4 (5 months), T5 (6 months), T6 (7 months), T7 (11 months) and T8 (13 months).

**Table 4**

| **Strains - ID** | **Storage T** | **Time** | | | | | | | | |
|---|---|---|---|---|---|---|---|---|---|---|
| | | **T0** | **T1** | **T2** | **T3** | **T4** | **T5** | **T6** | **T7** | **T8** |
| | | **Log₁ₚ (CFU/g)** | | | | | | | | |
| ***P. kudriavzavii*** | **Room** | **9.34** | **914±0.06^{L}** | **9.74±0.24^{a}** | **8.27±0.12** | **8.25±0.12^{b}** | **8.47±0.07^{b}** | **8.3±0.09^{b}** | **6.6±0.03^{c}** | **0^{c}** |
| | **Refrig.** | **9.34** | **9.32±0.04^{b}** | **9.41±0.06⁴** | **9.26±0.16^{a}** | **9.25±0.07^{a}** | **9.34±0.04^{a}** | **9.52±0.05^{a}** | **9.39±0.04^{c}** | **9.26±0.004^{b}** |
| | **Freezer** | **9.34** | **9.52±0.05^{a}** | **9.52±0.03^{a}** | **0 28±0 06^{a}** | **9.38±0.05^{a}** | **9.43±0.03^{a}** | **9.54±0.05^{a}** | **9.5±0.02^{a}** | **9.48±0.02^{b}** |
| **L. *plantarum*** | **Room** | **11.43** | **10.96±0.01^{b}** | **10.07±0.09^{b}** | **9.58±0.07^{b}** | **9.5±0.04^{c}** | **928±0.05^{a}** | **8.83±0.04^{b}** | **0^{b}** | **0^{c}** |
| | **Retng.** | **11.43** | **11.04±0.07^{ab}** | **11.05±0.11^{b}** | **10.74±0.04^{a}** | **10.3±0.1^{a}** | **10.52±0.01^{b}** | **10.09±0.08^{a}** | **10±0ₐ** | **9.92±0,04^{b}** |
| | **Freezer** | **11.43** | **11.16±0.11^{a}** | **11.08±0.06^{a}** | **10.69±0.06^{a}** | **10.48±0.03^{b}** | **10.47±0.04^{b}** | **10.1±0.05^{a}** | **10±0^{a}** | **10.16±0.05^{a}** |
| ***A.malorum*** | **Room** | **8.53** | **0^{c}** | **0^{b}** | **0^{c}** | **5.39±0.08^{b}** | **0^{b}** | **0^{b}** | **0^{c}** | **0^{a}** |
| | **Refng.** | **8.53** | **1.12±0.13^{p}** | **8.35±0.11^{a}** | **7.93±0.05^{b}** | **8.18±0.13^{a}** | **8.3±0.0.12^{a}** | **8.45±0.04^{b}** | **7.41±0.08^{b}** | **7.51±0.12^{b}** |
| | **Freezer** | **8.53** | **8.72±0.09^{a}** | **8.27±0.06^{a}** | **8.38±0.09^{a}** | **8.3±0.08^{a}** | **8.48±0.03^{a}** | **8.57±0.09^{a}** | **7.92±0.04^{b}** | **0^{a}** |

### 3. Back-slopping (BS) at laboratory scale: yeasts, LABs and AABs

### 3.1 Growth tests on pasteurized cocoa pulp

Cocoa pulp provided by Soremartec S.A was tested as a growth substrate for the identified best-performing microorganisms.

Preliminary trials on pulp were carried out by inoculating at 1% the LAB strain *L. plantarum* (DSM 35385) and the yeast strain *P. kudriavzevii* (DSM 35384) separately. Trials were performed in triplicate on undiluted pulp (TQ) and on pulp diluted at 1:5 and 1:10 (w/w) ratio with sterile distilled water.

The *L. plantarum* strain was inoculated in Pyrex of 100mL in 100mL of pulp (anaerobic conditions) as 10⁷ CFU/g, while the *P. kudriavzevii* strain was inoculated in a Pyrex of 250mL in 100mL of pulp (aerobic conditions) as 10⁶ CFU/g: 1mL of product was taken at time 0, when the pulp was inoculated, to verify that inoculation had taken place.

Dilutions and plate counts were also made at 24 and 48 h of incubation (room temperature) and after 7 and 14 days: the purpose was to monitor the growth and contamination of the inoculated product stored at room temperature. If the inoculated pulp will allow the growth of microorganisms even after two weeks, tests will continue until the end of microorganism growth is determined (i.e., until the post-plateau phase of microbial growth is reached, which has been considered to correspond to a minimum threshold of approximately in a range from 10⁵ to 10⁶ CFU/ml). At each time the pH was measured.

Table 3 below shows the pH values at 28°C of the three growth media at the different count times.

**Table 5**

| | | **Time (days)** | | | |
|---|---|---|---|---|---|
| | | **0** | **2** | **7** | **14** |
| **Y - pH at room temperature** | **TQ** | 3,35 | 3,23 | 3,42 | 3,4 |
| | **01:05** | 3,35 | 3,27 | 3,33 | 3,21 |
| | **01:10** | 3,35 | 3,29 | 3,43 | 3,36 |
| **LAB - pH at room temperature** | **TQ** | 3,35 | 3,91 | 3,71 | 3,54 |
| | **01:05** | 3,35 | 3,88 | 3,46 | 3,21 |
| | **01:10** | 3,35 | 3,77 | 3,43 | 3,12 |

Figure 1 shows the *P. kudriavzevii* strain growth curves in pulp as-is (TQ - blue), pulp diluted 1:5 (green) and pulp diluted 1:10 (orange) measured at 0-24-48-168-336-672 hours.

Figure 2 shows the *L. plantarum* strain growth curves in pulp as-is (TQ - blue), pulp diluted *1:5* (green) and pulp diluted 1:10 (orange) measured at 0-24-48-136 hours.

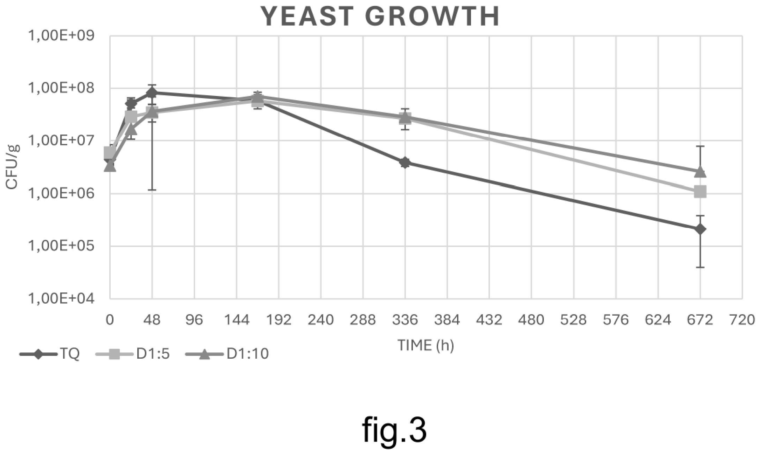

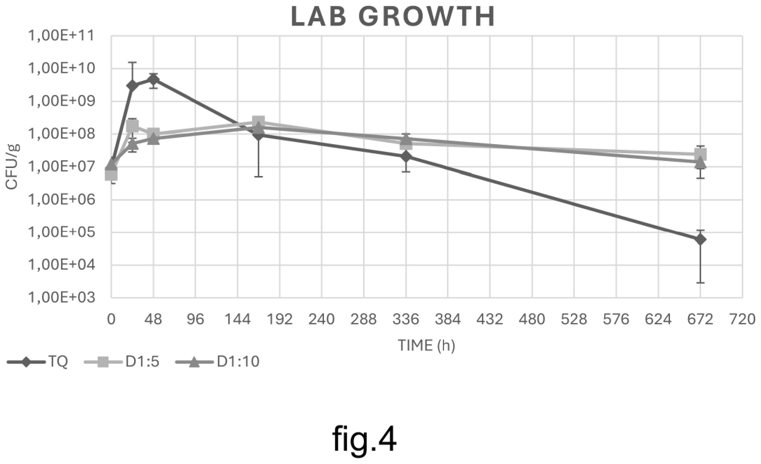

Both Figures 3 and 4 show a greater peak of growth in the short term (up to 48 hours) for the as-is pulp; on the contrary, in the long term there is a greater decrease in viable count. However, in the diluted pulp (with little difference between the two dilutions), the growth is maintained over the long term with a less pronounced decrease, and by only one log after 4 weeks for LAB.

Preliminary trials on pulp were also performed with the AAB strain, *A. malorum* (DSM 35383).

First of all, the strain was inoculated at 1% in a 250mL Pyrex with 100mL of undiluted pulp (TQ) and on pulp diluted at 1:2 and 1:5 (w/w) ratio (aerobic conditions), with sterile distilled water. Trials were performed in triplicate. 1mL of product was taken at time 0, when the pulp was inoculated, to verify that inoculation had taken place. Dilutions and plate counts were also made at 24 and 48 h of incubation (room temperature) and after 7 and 14 days: the purpose was to monitor the growth and contamination of the inoculated product stored at room temperature. Unfortunately, after one week, the growth decreases drastically, leaving only a few viable cells.

Subsequently, the AAB strain was inoculated at 1% in a final volume of 100mL in a 250mL Pyrex with different concentrations of Ethanol and two different types of EtOH: EtOH absolute anhydrous (Carlo Erba, code: 414607) and neutral EtOH of agricultural origin (Distillerie Vener, Parma, Italy). The best solution was 2% (w/w) of neutral EtOH of agricultural origin (Distillerie Vener, Parma, Italy).

All these data are important in determining the conditions for next back-slopping experiments.

### 3.2 Cocoa pulp as carrier for selected strains potentially useful as starters: general process

The purpose of this test was to develop a protocol that allows cocoa pulp to be used as an inoculation medium for cocoa pulp over an extended period of time. The goal was to prevent contamination while maintaining a stable and uniform inoculum.

Each freeze-dried strain (DSM 35384; DSM 35385; DSM 35383) was inoculated at 1%, i.e. the minimum inoculum in which an effect had already been observed, into a certain amount of pasteurized cocoa pulp (steps a) to c) of the process of the invention). After a predetermined growth time (in this study the time was standardized for all the microorganisms at 48h at 30°C for yeast and acetic acid bacteria and 37°C for lactic acid bacteria), a high-density culture was obtained (i.e. the "mother culture of fermented pulp") (step d) of the process of the invention). The mother culture of fermented pulp was divided in 3 aliquots:
i. the first aliquot being used as a fermentation inoculum for pasteurized fresh cocoa pulp to obtain a "first fermented pulp" (i.e. step e) of the process of the invention), whereafter subsequent steps of inoculating a freshly prepared pasteurized cocoa pulp with an aliquot of the fermented pulp obtained from the corresponding preceding step were performed (i.e. step f) of the process of the invention), thereby shortening the initial phase of fermentation, reducing the risk of fermentation failure and resulting in the promotion of preferable changes during the process;
ii. the second aliquot being inoculated (at 1 - 1.5%) in another stock of freshly prepared pasteurized cocoa pulp (if a fresh inoculum is required)
iii. the third aliquot being stored to be inoculated later (after days or weeks) in another stock of freshly prepared pasteurized cocoa pulp.

The mechanism of this process is the same for all three microorganisms and it could be repeated at different times, to determine the dominance of the best-adapted strains.

The specifics of the laboratory tests are detailed in the following of the present disclosure.

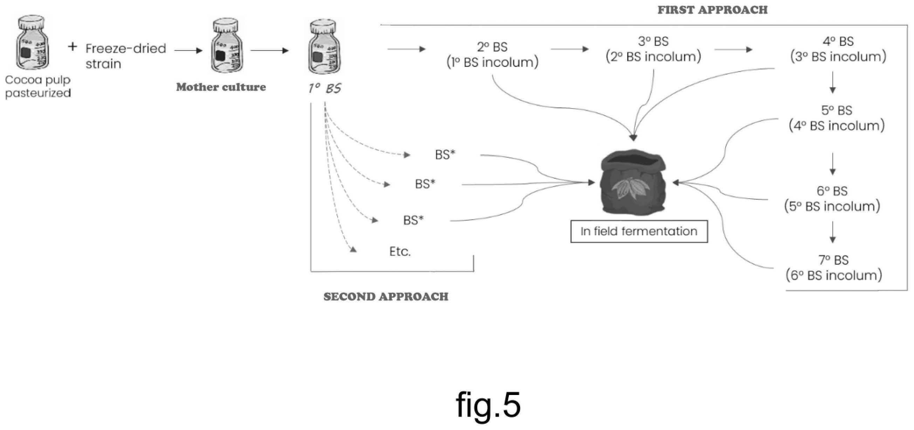

To make the most of the initial inoculation of freeze-dried culture into pulp, a first approach or a second approach may be used. A schematic overview of such first approach and second approach is provided in Figure 5.

The first approach involves starting from the "mother culture of fermented pulp" (i.e. the pulp inoculated with the freeze-dried starter culture) to create the "first fermented pulp" (1st BS). Then, an aliquot from the "first fermented pulp" (also designated as "1st BS") is used to inoculate pasteurized-fresh-pulp to obtain the "second fermented pulp" (also designated as "2nd BS"). The first fermented pulp aliquot employed corresponds to 1% of the total pasteurized-fresh-pulp to be inoculated. From the "second fermented pulp" (2nd BS), immediately after a 48-hour incubation or after a short-storage in the refrigerator (4°C), an aliquot is taken to create a "third fermented pulp" (3rd BS). Similarly, from the "third fermented pulp" (3rd BS), immediately after incubation or after a short refrigeration period, another aliquot is taken to create a "fourth fermented pulp" (4th BS). This process is repeated n times, until the inoculum remains clean and stable over time. The first approach is appropriate when it is necessary to inoculate fermentations at short intervals.

The second approach involves starting from the "mother culture of fermented pulp" (i.e. the pulp inoculated with the freeze-dried starter culture) to create the "first fermented pulp" (1st BS). Rather than being immediately used to create the nth fermented pulps (nth BS) as described with reference to the first approach, this approach involves storing the "first fermented pulp" (1st BS) in the refrigerator for a period of time which depends on the inoculated microorganism - e.g., for LAB, it can last up to 12 weeks. During this storage period, aliquots of the stored "first fermented pulp" (1st BS) are used as the starting inoculum for the fermentation of freshly prepared pasteurized pulp, thereby creating respective "second fermented pulps" (2nd BS). Then, the back-slopping steps are repeated to create the nth fermented pulps (nth BS) as described with reference to the first approach. The second approach is appropriate when cocoa fermentations are spaced out over time and fresh inoculum is not needed daily.

Both in the first approach and in the second approach, a first aliquot of the "first fermented pulp" (1st BS) and/or of any of the n fermented pulps subsequently obtained (nth BS) is added to cocoa beans for in-field fermentation to obtain a cocoa bean fermentation product.

It is interesting to consider the intersection of the two approaches. Starting from the 1st BS, after the incubation period, it can be split into two aliquots: the first one, following the second approach, will be stored in the refrigerator; the second one will be used to inoculate fresh-pasteurized-pulp (2nd BS), following the first approach described above. The 2nd BS, after the incubation period, can in turn be split into two parts, each following the two approaches respectively. In this way, a new stock of inoculated pulp is created in the refrigerator and a 3rd BS is ready to be used in the field. This intersection ensures that there are always "fresh" stocks in the refrigerator as well as stocks ready for use in the field.

### 3.3 Back-slopping at laboratory scale: testing of multiple storage conditions and the BS technique

For the laboratory trials, a detailed work plan was organized to test multiple storage conditions and the BS technique.

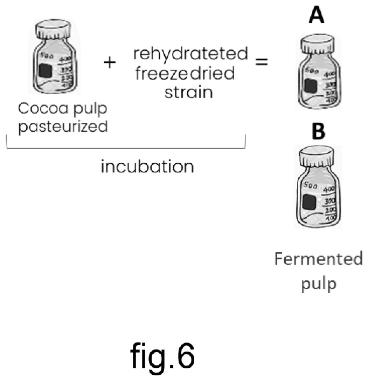

To start, 1g of each freeze-dried strain (DSM 35384; DSM 35385; DSM 35383) was rehydrated in 9mL of a 9% NaCl solution and inoculated at 1% into fresh-pasteurized-pulp placed in two different Pyrex bottles **(A, B),** each filled with 100mL of that pulp (Figure 6). A 250mL Pyrex bottle was used for yeast and AAB strains, to guarantee anaerobiosis conditions of growth. A 100mL Pyrex bottle was used for LAB strain, to guarantee aerobiosis environment. The inoculum vitality was assessed by performing triplicate counts on the inoculated solution. The Pyrex bottles were incubated at 30°C for yeast and AAB for 48 hours, with counts performed at 0, 24, and 48 hours, in triplicate.

Bottle **A** was stored in a refrigerator to serve as the "mother culture" for the BS experiments.

Bottle **B** was placed in the refrigerator and served as a control, remaining unopened. This setup enables the assessment of whether repeated temperature fluctuations and air exposure, occurring each time Bottle C (see below) is opened to extract an aliquot for inoculation, affect the viability of the microorganisms.

Subsequently, two distinct laboratory approaches were adopted to cover a wider range of scenarios and better simulate field conditions. Strain vitality in Bottle **A** stored in the refrigerator was assessed after 1, 2 and 4 weeks. Both approaches were started only after assessing the viability of the strains fermented in pulp following one week of refrigeration storage. If vitality had significantly decreased, adjustments would have been needed to make the pulp environment more suitable for growth before proceeding with further trials.

### 3.3.1 First approach

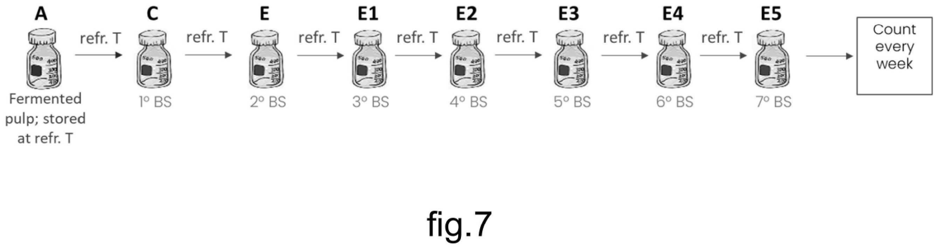

In the first approach (schematically illustrated in Figure 7), 1mL of pulp from Bottle **A** was used to inoculate 100mL of pulp in a Pyrex bottle (hereafter referred to as **C**-1°BS) after one week of refrigeration. **C** was incubated at 30°C (Yeast and AAB) or 37°C (LAB) for 48 hours, with growth counts performed at 0, 24, and 48 hours of incubation.

In all experiments disclosed in the present description, 250 ml Pyrex bottles were employed for yeasts and AABs, to guarantee anaerobiosis growth conditions, while 100 ml Pyrex bottles were employed for LABs, to guarantee aerobiosis growth conditions.

**C** was then stored at refrigerator temperature, and after one week, microorganisms viability counts were conducted to assess its persistence and the inoculum vitality for the next fermentation, referred to as **E** (100mL of pulp in a Pyrex bottle) in Figure 7. **E** was incubated at 30°C (yeast and AAB) or 37°C (LAB) for 48 hours, with counts taken at 0, 24, and 48 hours.

From **E,** after one week of refrigeration, 1mL was taken and used to inoculate **E1** (100mL of pulp in a Pyrex bottle). **E1** was incubated at 30°C (yeast and AAB) or 37°C (LAB) for 48 hours, with vitality counts performed at 0, 24, and 48 hours.

From **E1**, after one week of refrigeration, 1mL was taken and used to inoculate **E2** (100mL of pulp in a Pyrex bottle). **E2** was incubated at 30°C (yeast and AAB) or 37°C (LAB) for 48 hours, with vitality counts performed at 0, 24, and 48 hours.

From **E2,** after one week of refrigeration, 1mL was taken and used to inoculate **E3** (100mL of pulp in a Pyrex bottle). **E3** was incubated at 30°C (yeast and AAB) or 37°C (LAB) for 48 hours, with vitality counts performed at 0, 24, and 48 hours.

From **E3,** after one week of refrigeration, 1mL was taken and used to inoculate **E4** (100mL of pulp in a Pyrex bottle). **E4** was incubated at 30°C (yeast and AAB) or 37°C (LAB) for 48 hours, with vitality counts performed at 0, 24, and 48 hours.

From **E4,** after one week of refrigeration, 1mL was taken and used to inoculate **E5** (100mL of pulp in a Pyrex bottle). **E5** was incubated at 30°C (yeast and AAB) or 37°C (LAB) for 48 hours, with vitality counts performed at 0, 24, and 48 hours.

In Table 6 for yeast, Table 7 for LAB and Table 8 for AAB the counts of BS E1, E2, E3, E4 and E5 at time 48h, i.e. at the end of each fermentation, are reported.

### Results

In the following Tables, Y stands for "yeast" (DSM 35384), L stands for "lactic acid bacteria" (DSM 35385), and A stands for "acetic acid bacteria" (DSM 35383). The identification letters of the bottles are also shown.

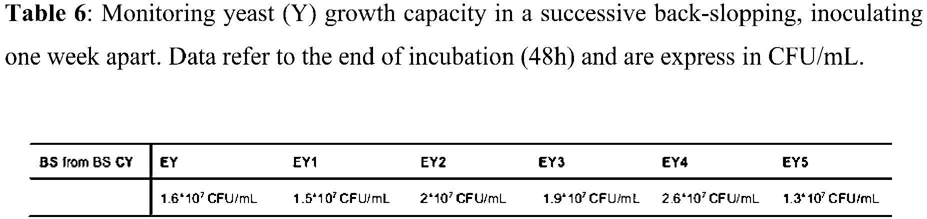

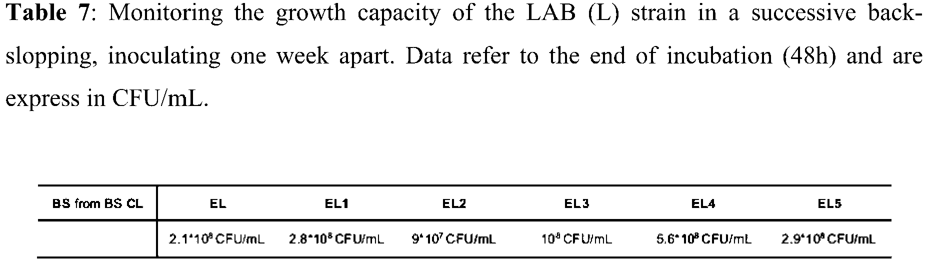

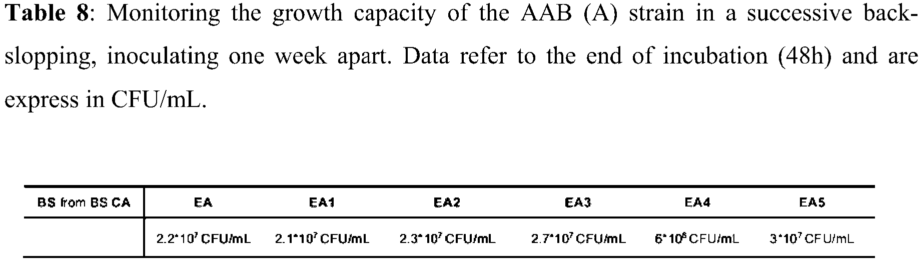

Since the system successfully supported 7 consecutive BS for the yeast and LAB strains, it was decided to continue monitoring the 7th BS for 5 weeks under refrigeration conditions (Table 9 and Table 10).

Table 9 and Table 10 include the 5-week monitoring of the 7th BS as well as the monitoring of the 3rd and 4th BS after 1, 2, and 3 weeks of storage.

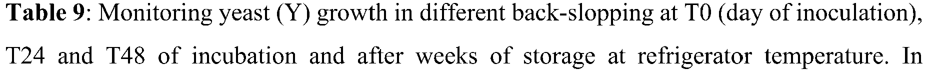

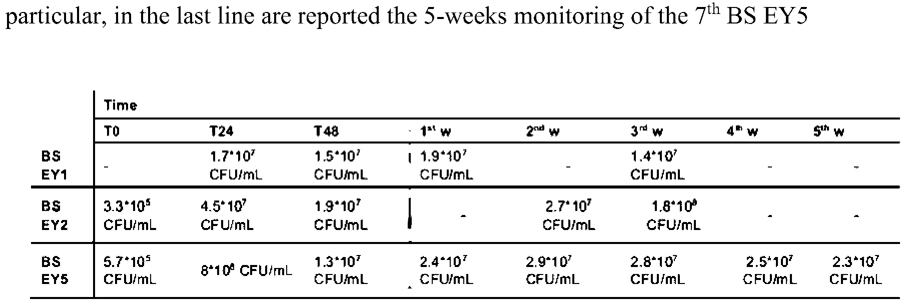

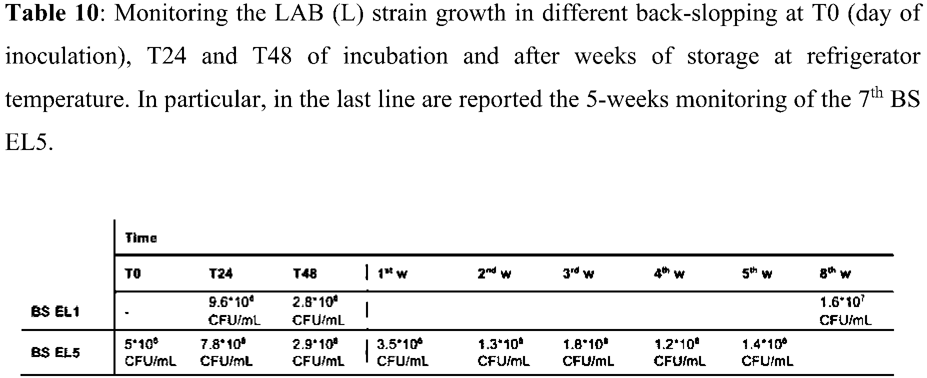

### 3.3.2 Second approach

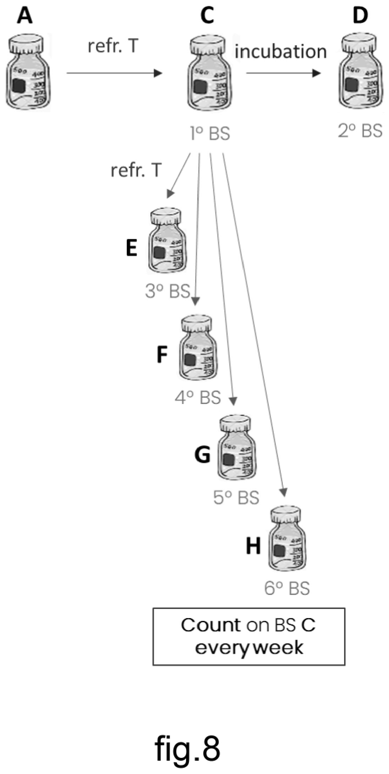

In the second approach (schematically illustrated in Figure 8), 1mL of pulp from Bottle **A** was used to inoculate 100mL of pulp in a Pyrex bottle (hereafter referred to as **C)** after one week of refrigeration. **C** was incubated at 30°C (yeast and AAB) and 37°C (LAB) for 48 hours, with growth counts performed at 0, 24, and 48 hours of incubation.

After the 48-hour incubation, 1mL of **C** was immediately used to initiate a new fermentation, named as **D** (100mL of fresh-pasteurized-pulp; Pyrex bottle), which was also incubated at 30°C (for yeast and AAB) and 37°C for LAB, for 48 hours. Vitality counts were performed in triplicate at 0, 24, and 48 hours.

**C** was then stored in the refrigerator, and after one week, strain vitality counts were conducted to assess its persistence and the inoculum capacity for the next fermentation, referred to as **E** (100mL of pulp in a Pyrex bottle). **E** was incubated at 30°C (for yeast and AAB) or 37°C for LAB for 48 hours, with counts taken at 0, 24, and 48 hours.

The purpose of using **C** to create both **D** and **E** at different time intervals was to evaluate whether there are differences in growth when the inoculum is used immediately after fermentation or after one week of storage. Results of the two fermentations are reported in Table 11 for yeast, Table 12 for LAB and Table 13 for AAB.

According to the second approach, **C** was then used every week to inoculate new BS. More specifically, each week, 1mL of **C,** stored in the refrigerator, was taken and inoculated into 100mL of clean pasteurized pulp placed in a Pyrex bottle. The subsequent BS are identified in Figure 8 as **F** (after 2 weeks of storage), **G** (after 3 weeks), and **H** (after 4 weeks). All were incubated at 30°C (yeast and AAB) and 37°C (LAB) for 48 hours, with counts performed at 0, 24, and 48 hours of incubation. In Table 14 for yeast, Table 15 for LAB and Table 16 for AAB are reported the counts of F, G and H fermentations at time 48h, so at the end of each fermentation.

After 4 weeks, since the method was successful, it was decided to continue weekly counting of **C,** the inoculum, and evaluate its shelf life at refrigerator temperature, without wasting pulp by continuing fermentation. The counts were carried out for up to 12 weeks (data are reported in the first line of Table 17 for yeast, Table 18 for LAB and Table 19 for AAB), a period considered more than sufficient to meet the field trial requirements. Tables 17, 18 and 19 also include the monitoring of BS G and H after some weeks of storage in the refrigerator.

### Results

In the following Tables, Y stands for "yeast" (DSM 35384), L stands for "lactic acid bacteria" (DSM 35385), and A stands for "acetic acid bacteria" (DSM 35383). The identification letters of the bottles are also shown.

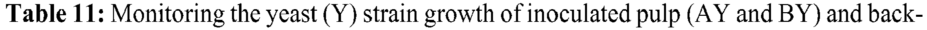

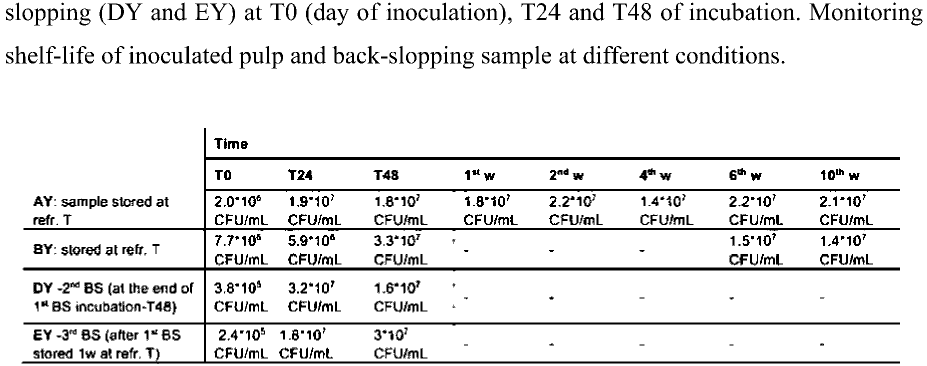

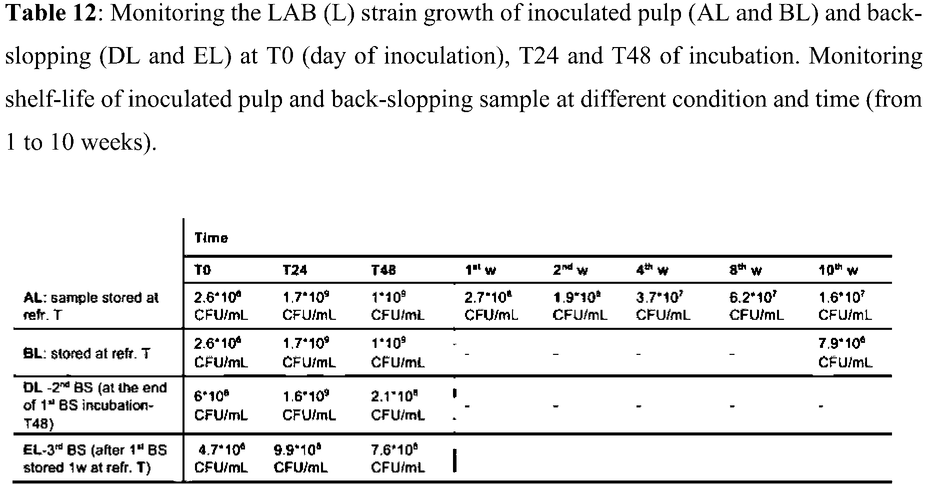

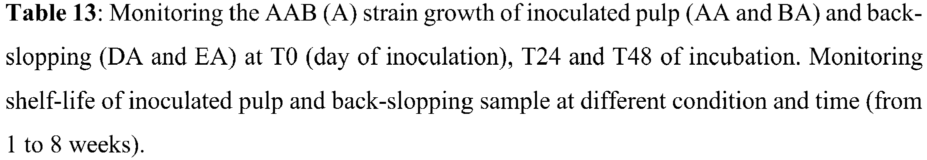

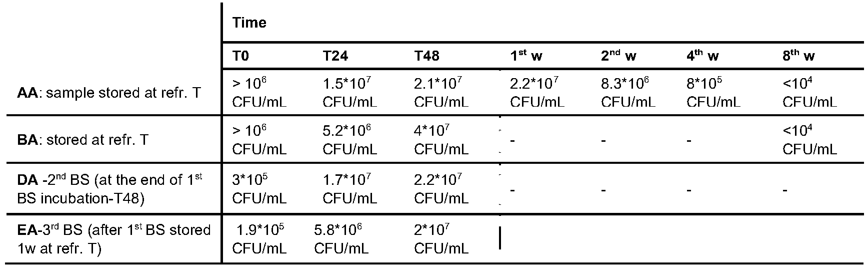

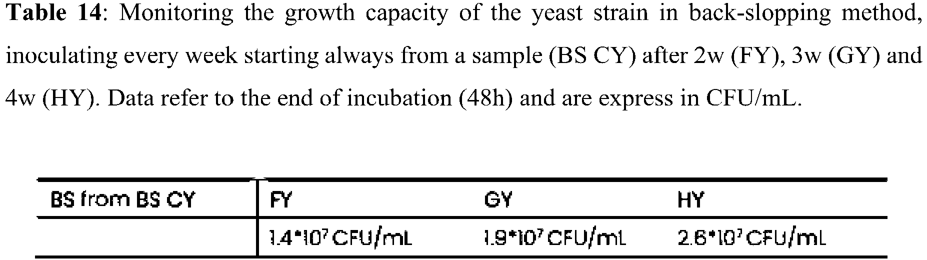

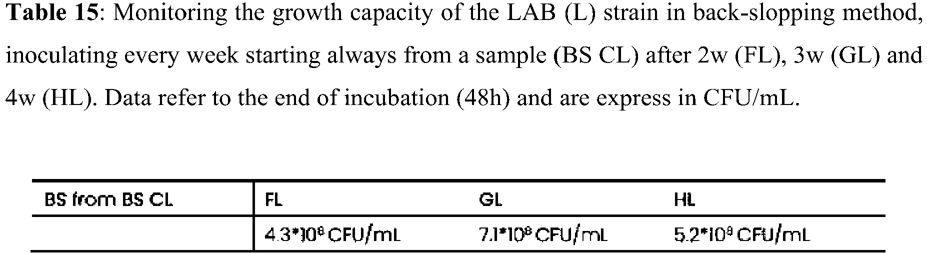

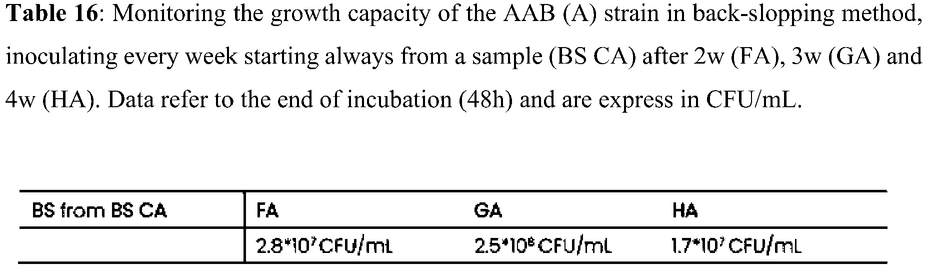

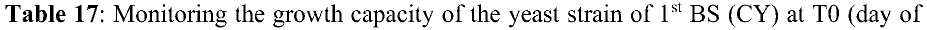

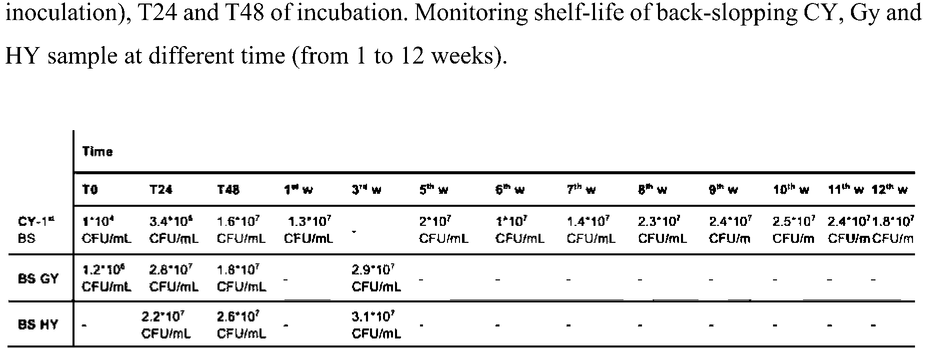

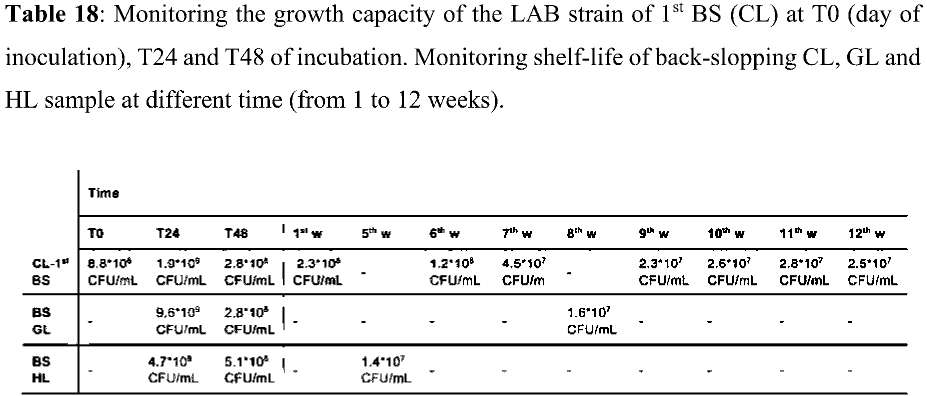

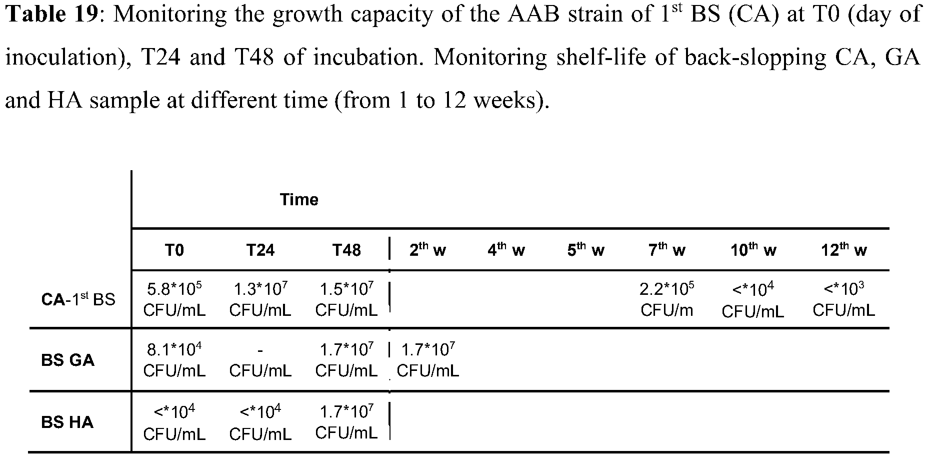

### 4. In-field fermentation

The fermentation trials were conducted in farm facilities in Ecuador. The fermentation was conducted for 6 days in heaps covered with jute sacs and black plastic films, which were flipping and mixed every 2 days. Each heap contained 120 kg of cacao beans, and the variety used was the hybrid CCN51. After harvesting, the pods were opened manually and then mechanically de-pulped before starting the fermentation as its usually performed by the supplier for this variety.

The trials were designed as shown in Table 20, using 2 yeast strains (one of which was DSM 35384), 2 LAB strains (one of which was DSM 35385) and 2 AAB strains (one of which was DSM 35383) in various combinations. To mimick what occurs in spontaneous fermentation and the natural succession of microorganisms, the yeasts were inoculated at the beginning of fermentation (day 1), the LABs on day 3 and the AABs on day 5. All fermentations were conducted for 6 days until completing the process and then the piles were opened and spread to allow natural drying.

**Table 20: trials description and application scheme**

| **FERMENTATION TRIAL** | **Yeast a** | **Yeast b** | **LAB a** | **LAB b** | **AAB a** | **AAB b** |
|---|---|---|---|---|---|---|
| **F1** | | | | | | |
| **F2** | | | | | | |
| **F3** | | | | | | |
| **F4** | | | | | | |
| **F5** | | | | | | |
| **F6** | | | | | | |
| **F7** | | | | | | |
| **F8** | | | | | | |
| **F9** | | | | | | |
| **F10** | | | | | | |
| **F11** | | | | | | |
| **F12** | | | | | | |
| **F0 (Control)** | | | | | | |
| **FTT (Control)** | | | | | | |

To reactivate the freeze-dried strains, 2.5 grams of sample were rehydrated in a 9% NaCl solution at a 1:10 ratio. This solution was used to inoculate 1 L of pasteurized fresh pulp at a 1% concentration, which was then incubated at the appropriate temperature (depending on the microorganism used) for 48 hours. Subsequently, the resulting starter culture was stored in the refrigerator to initiate the BS process previously described.

### 4.1 DNA sampling and extraction

Samples were taken at different times (T0-T1-T2-T3) during the fermentation process, as represented in Table 21. Beans were collected from the fermenting pile in 3 different positions, top- middle and bottom-, and placed in yellow cap tubes. Then 5 ml of distilled sterilized water were added to each tube to wash off the pulp and DNA samples from the microorganisms growing in the surface of the beans. The tubes were then vortexed for 2 minutes each and let to rest for 10 minutes, after that time and before taking the supernatant sample, they were vortexed again for 2 minutes to enhance the surface wash. Then with a micropipette 75 µl of the supernatant was taken from each tube and placed in the FTA^{™} (Flinders Technology Associates) Card, Whatman (Supplier: QIAGEN).

**Table 21: sampling scheme**

| **FERM - DAY1** | **FERM - DAY2** | **FERM - DAY3** | **FERM - DAY4** | **FERM - DAY5** | **FERM - DAY6** |
|---|---|---|---|---|---|
| Bean Mixing | | Bean Mixing | | Bean Mixing | |
| T0 - FTACard | | T1 - FTACard | | T2 - FTACard | T3 - FTACard |

The FTA^{™} GeneCards were let dry at environmental conditions, and then placed in protective envelopes and stored at ambient temperature.

Flinders Technology Associates (FTA) matrix cards developed by Whatman allows rapid collection, purification, and analysis of genetic material from a wide range of sources.

FTA^{™} GeneCards are designed for long-term storage at room temperature of DNA samples from a wide variety of biological samples that include bacterial cultures, blood, buccal cells, tissue, microorganisms, and plant tissue. The DNA stored in these Cards is ready for PCR amplification, as the cards are impregnated with a chemical that lyses cell membranes and denatures proteins upon contact. The nucleic acids are immobilized and protected from UV damage and microbial and fungal attack. To use FTA cards, the sample is applied as a liquid or pressed in the surface, then let dry at room temperature (Qiagen).

According to the manufacturer's protocol (Qiagen, n.d.), DNA was extracted by the FTA^{™} GeneCards through Purification Reagent washing and TE⁻¹ Buffer (10 mM TRIS; 0,1 mM EDTA; pH8) solution.

### 4.2 Results

The inventors assessed the temporal dynamics of the microbial population, divided into bacteria (BAC) and yeasts (ITS), across the 12 fermentations inoculated with the different microorganisms, along with the two control fermentations (F0 and FTT), which represent spontaneous fermentations.

The first notable observation was the clear difference between the two controls, which exhibited a succession of different microorganisms over time. This further highlights the completely spontaneous and non-standardized nature of cacao fermentation, as it is driven by a highly variable cacao microbiota.

Samples were collected at four time points:
- **T0:** At the beginning of fermentation, before any inoculation.
- **T1:** After 48 hours of fermentation, following yeast inoculation and before lactic acid bacteria inoculation. This time point allows the evaluation of the dynamics of the inoculated yeasts and their influence on the microbiota.
- **T2:** After 96 hours of fermentation, following lactic acid bacteria inoculation and before the inoculation of acetic acid bacteria. This time point helps assess the influence of bacteria (if inoculated) and how the yeasts have shaped the subsequent fermentation step.
- **T3:** After 120 hours of fermentation, to evaluate the influence of acetic acid bacteria, if inoculated.

### BAC analysis

The microbial composition at T1 appears to be different, with higher presence of multiple genera, especially in inoculated fermentations.

*Bacillus* is the dominant genera, particularly in control fermentations (F0_t1 and FTT_t1), but there is a greater presence of other bacterial genera in inoculated fermentations. More specify, in inoculated fermentations there is a noticeable presence of *Limosilactobacillus, Lactiplantibacillus,* and *Pseudomonas.* These variations suggest the initial response of the microbial community to yeast inoculation before T1.

The higher presence of *Limosilactobacillus* and *Lactiplantibacillus* at T1 suggests that lactic acid bacteria were more active early in the fermentation, but may be outcompeted later.

*Acetobacter* and *Gluconobacter* are present in certain fermentations, but at low proportion, compared to other genus. The exception of F3, in which two yeast were inoculated, with a significant presence of *Gluconobacter* could indicate a specific alteration compared to the other analysed fermentations. *Gluconobacter* is a genus of acetic acid bacteria known for its ability to oxidize sugars and ethanol into acetic acid, which can affect the sensory profile of the final product.

Finally, in T1 there was a high proportion of low-abundance or unidentified bacteria in the early phase of fermentation.

Biological and Fermentation Implications:
- The increase in microbial diversity at T1 suggests that the microbial community is still stabilizing.
- The presence of lactic acid bacteria (*Limosilactobacillus, Lactiplantibacillus)* at this stage suggests that fermentation is transitioning towards the dominance of acid-producing bacteria.
- The lower presence of acetic acid bacteria at T1, except for F3, aligns with their expected later-stage activity when oxygen becomes more available.

The variability among inoculated fermentations supports the idea that yeast inoculation influences community dynamics early on, but spontaneous microbial interactions also play a significant role.

*Bacillus* was the most prevalent genus across nearly all samples in T2, especially in the control fermentations (F0_t2 and FTT_t2), where it dominated almost entirely. Also in F4 and F5 there was an high prevalence of *Bacillus* genus.

*Lactiplantibacillus* and *Limosilactobacillus* appeared in various fermentations. Particularly in F7-F8 and F5, where was inoculated. A significant presence was also observed in fermentation F3, where only two yeasts were inoculated, suggesting their role in creating an environment suitable for its growth. *Lactiplantibacillus,* on the other hand, was found in F9 and F6, two fermentations where it was inoculated with either a single yeast or two, respectively. This suggests that at T2, the presence of only one yeast allows the dominance of a lactic acid bacteria rather than an acetic acid bacteria, as seen in F6, where the *Acetobacter* genus is clearly evident.

Some inoculated fermentations (e.g., F3_t2, F2_t2) exhibited a more balanced presence of different bacterial genera, indicating a shift in community structure due to inoculation.

The differences between control (in particular F0) and inoculated fermentations highlight the impact of microbial inoculation on bacterial dynamics. The variation in microbial communities across fermentations supports the idea that cacao fermentation is highly variable and dependent on initial microbial composition and environmental factors.

Bacillus had become the dominant genus across nearly all fermentations at T3, as in F7, F6, F5, F4, F3, F12, F10 and in F0. This trend was already present in T2, but at T3, *Bacillus* was even more prevalent, suggesting its strong persistence in the later fermentation stages.

Compared to T1 and T2, there was a noticeable decrease in *Lactiplantibacillus, Acetobacter,* and *Limosilactobacillus,* with the exception of fermentations F11 and F12, where the *Acetobacter* genus was clearly present. This suggests that lactic acid bacteria and acetic acid bacteria, which were more present in earlier stages, in T3 has been outcompeted or had completed their functional role in fermentation.

In F0_t3 and FTT_t3 (spontaneous fermentations), *Bacillus* and *Dickeya* dominated almost entirely, with only minor contributions from other genera for F0.

Some fermentations still contained *Pseudomonas* (F8), *Streptomyces* (F2, F3), and *Komagataeibacter,* but at very low relative abundance, indicating they might not play a major role at this stage.

Comparison of bacteria composition across Time Points (T1, T2, T3):
- T1 (48h): More diverse bacterial composition, with a significant presence of *Lactiplantibacillus, Limosilactobacillus, and Gluconobacter* alongside *Bacillus.*
- T2 (96h): *Bacillus* starts becoming dominant, but lactic acid bacteria and acetic acid bacteria are still present in several fermentations, which play essential roles in acidifying the environment.
- T3 (120h): *Bacillus* remain one of the dominant genus, suggesting its ability to outcompete other bacteria and maintain dominance in the final stage of fermentation, due to environmental conditions such as pH, oxygen levels and nutrient availability.
- The decline of lactic acid and acetic acid bacteria, over time, may indicate that their metabolic contributions (such as acid production) have stabilized the fermentation process.
- The differences between control and inoculated fermentations suggest that inoculation may influence microbial succession. The data further confirm the idea that cacao fermentation is highly dynamic and still non-standardized process.

### ITS analysis

Since yeasts dominate only the first 24-48 hours of cocoa fermentation and are therefore inoculated at the beginning, tracking their evolution over time, as done for bacteria, is not particularly meaningful, although it is possible. For this reason, only T1 is presented, limited to cases where it was possible to study the yeast phase dynamics.

*Hanseniaspora* and *Millerozyma* were the most abundant genera across almost all fermentations. This aligns with the common trend in cacao fermentation, where *Hanseniaspora* is typically the dominant yeast in early fermentation due to its rapid growth and ability to thrive in the initial sugar-rich environment, producing ethanol.

Genera such as *Lachancea, Ogataea, Saccharomyces,* and *Wickerhamom* were more and differently represented in certain inoculated fermentations, indicating potential competition among yeasts. The presence of other yeast genera in inoculated fermentations also suggests potential microbial interactions that might affect fermentation performance and final cacao quality. These yeasts may have had an impact, but did not fully take over the fermentation process, as could be in some other cacao fermentations.

The presence of unclassified ("Not Assigned") and "Others" categories suggests that some yeasts could not be taxonomically resolved at the genus level, indicating microbial complexity.

### REFERENCES

Arroyo-lópez, F. N., Salvadó, Z., Tronchoni, J., Guillamón, J. M., Barrio, E., & Querol, A. (2010). Susceptibility and resistance to ethanol in Saccharomyces strains isolated from wild and fermentative environments. Yeast, 27(12), 1005-1015. https://doi.org/10.1002/yea.1809 Castro-Alayo, E. M., Idrogo-V Asquez, G., Ul Siche, R., & Cardenas-Toro, F. P. (2019). Formation of aromatic compounds precursors during fermentation of Criollo and Forastero cocoa. Forastero Cocoa. Heliyon, 5, 1157. https://doi.org/10.1016/j.heliyon.2019
Cempaka, L., Aliwarga, L., Purwo, S., & Penia Kresnowati, M. T. A. (2014). Dynamics of cocoa bean pulp degradation during cocoa bean fermentation: Effects of yeast starter culture addition. Journal of Mathematical and Fundamental Sciences, 46(1), 14-25. https://doi.org/10.5614/j.math.fund.sci.2014.46.1.2
Chen, L., Yang, J., Yu, J., Yao, Z., Sun, L., Shen, Y., & Jin, Q. (2005). VFDB: A reference database for bacterial virulence factors. Nucleic Acids Research, 33(DATABASE ISS.). https://doi.org/10.1093/nar/gki008
Copetti, M. V., Iamanaka, B. T., Pitt, J. I., & Taniwaki, M. H. (2014). Fungi and mycotoxins in cocoa: From farm to chocolate. In International Journal of Food Microbiology (Vol. 178, pp. 13-20). Elsevier B.V. https://doi.org/10.1016/j.ijfoodmicro.2014.02.023
De Vuyst, L., & Weckx, S. (2016). The cocoa bean fermentation process: from ecosystem analysis to starter culture development. In Journal of Applied Microbiology (Vol. 121, Issue 1, pp. 5-17). Blackwell Publishing Ltd. https://doi.org/10.1111/jam.13045
Diaz-Muñoz, C., & De Vuyst, L. (2022). Functional yeast starter cultures for cocoa fermentation. In Journal of Applied Microbiology (Vol. 133, Issue 1, pp. 39-66). John Wiley and Sons Inc. https://doi.org/10.1111/jam.15312
Figueroa-Hernández, C., Mota-Gutierrez, J., Ferrocino, I., Hernández-Estrada, Z. J., González-Ríos, O., Cocolin, L., & Suárez-Quiroz, M. L. (2019). The challenges and perspectives of the selection of starter cultures for fermented cocoa beans. In International Journal of Food Microbiology (Vol. 301, pp. 41-50). Elsevier B.V. https://doi.org/10.1016/j.ijfoodmicro.2019.05.002
Gutiérrez-Ríos, H. G., Suárez-Quiroz, M. L., Hernández-Estrada, Z. J., Castellanos-Onorio, O. P., Alonso-Villegas, R., Rayas-Duarte, P., Cano-Sarmiento, C., Figueroa-Hernández, C. Y., & González-Rios, O. (2022). Yeasts as Producers of Flavor Precursors during Cocoa Bean Fermentation and Their Relevance as Starter Cultures: A Review. In Fermentation (Vol. 8, Issue 7). MDPI. https://doi.org/10.3390/fermentation8070331
Ho, V. T. T., Zhao, J., & Fleet, G. (2015). The effect of lactic acid bacteria on cocoa bean fermentation. International Journal of Food Microbiology, 205, 54-67. https://doi.org/10.1016/j.ijfoodmicro.2015.03.031
Jaimez, R. E., Barragan, L., Fernández-Niño, M., Wessjohann, L. A., Cedeño-Garcia, G., Cantos, I. S., & Arteaga, F. (2022). Theobroma cacao L. cultivar CCN 51: a comprehensive review on origin, genetics, sensory properties, production dynamics, and physiological aspects. In PeerJ (Vol. 9). PeerJ Inc. https://doi.org/10.7717/peerj.12676
Kongor, J. E., Hinneh, M., de Walle, D. Van, Afoakwa, E. O., Boeckx, P., & Dewettinck, K. (2016). Factors influencing quality variation in cocoa (Theobroma cacao) bean flavour profile - A review. In Food Research International (Vol. 82, pp. 44-52). Elsevier Ltd. https://doi.org/10.1016/j.foodres.2016.01.012
Ordoñez-Araque, R. H., Landines-Vera, E. F., Urresto-Villegas, J. C., & Caicedo-Jaramillo, C. F. (2020). Microorganisms during cocoa fermentation: Systematic review. Foods and Raw Materials, 8(1), 155-162. https://doi.org/10.21603/2308-4057-2020-1-155-162 Qiagen, F. (n.d.). FTA ® Cards. www.carlroth.com
Schwan, R. F., Cooper, R. M., & Wheals, A. E. (1997) Endopolygalacturonase secretion by Kluyveromyces marxianus and other cocoa pulp-degrading yeasts.
Viesser, J. A., de Melo Pereira, G. V., de Carvalho Neto, D. P., Favero, G. R., de Carvalho, J. C., Goés-Neto, A., Rogez, H., & Soccol, C. R. (2021). Global cocoa fermentation microbiome: revealing new taxa and microbial functions by next generation sequencing technologies. In World Journal of Microbiology and Biotechnology (Vol. 37, Issue 7). Springer Science and Business Media B.V. https://doi.org/10.1007/s11274-021-03079-2
Watson, A. L., & Chiu, N. H. L. (2016). Fluorometric cell-based assay for β-galactosidase activity in probiotic gram-positive bacterial cells - Lactobacillus helveticus. Journal of Microbiological Methods, 128, 58-60. https://doi.org/10.1016/j.mimet.2016.06.030 WO2009138419A2
Zankari, E., Hasman, H., Cosentino, S., Vestergaard, M., Rasmussen, S., Lund, O., Aarestrup, F. M., & Larsen, M. V. (2012). Identification of acquired antimicrobial resistance genes. Journal of Antimicrobial Chemotherapy, 67(11), 2640-2644. https://doi.org/10.1093/jac/dks261
Zhao, G., & Zhang, G. (2005). Effect of protective agents, freezing temperature, rehydration media on viability of malolactic bacteria subjected to freeze-drying. Journal of Applied Microbiology, 99(2), 333-338. https://doi.org/10.1111/j.1365-2672.2005.02587.x

## Claims

1. A process for cocoa bean fermentation comprising the steps of:
a) de-pulping cocoa beans to obtain cocoa pulp,
b) pasteurizing the cocoa pulp,
c) inoculating the pasteurized cocoa pulp with one or more freeze-dried fermentation starter cultures, wherein each of the one or more freeze-dried fermentation starter cultures comprises one or more microorganism strains selected from the group consisting of yeast strains, lactic acid bacteria (LAB) strains and acetic acid bacteria (AAB) strains,
d) incubating the inoculated pasteurized cocoa pulp for a time and at a temperature sufficient to obtain a mother culture of fermented pulp,
e) taking an aliquot of said mother culture of fermented pulp, inoculating pasteurized fresh cocoa pulp with said aliquot and incubating for a time and at a temperature sufficient to obtain a first fermented pulp,
f) repeating step e) for a number n of times, each time by inoculating a freshly prepared pasteurized cocoa pulp with an aliquot of the fermented pulp obtained from the corresponding preceding step to obtain a second, third and up to nth fermented pulp, until the cell count of the inoculated microorganism in the last obtained fermented pulp remains stable, and
g) adding a first aliquot of the first fermented pulp and/or of any of the fermented pulps subsequently obtained in step f), to cocoa beans for in-field fermentation and fermenting to obtain a cocoa bean fermentation product.

2. The process according to claim 1, further comprising taking a second aliquot or the remaining part of said first fermented pulp, optionally storing said second aliquot or remaining part at refrigerator temperature and, after the optional period of storage, using said second aliquot or remaining part as the starting inoculum for in-field cocoa bean fermentation.

3. The process according to claim 1 or 2, wherein a second aliquot of at least one or each of the fermented pulps created from step f), after an optional period of storage at refrigerator temperature, is used as an inoculum for in-field cocoa bean fermentation.

4. The process according to any of claims 1 to 3, wherein the inoculation of step c) is carried out by three subsequent freeze-dried starter culture inoculations, wherein the first inoculation is made with a freeze-dried starter culture comprising one or more yeast strains, the second inoculation is made with a freeze-dried starter culture comprising one or more LAB strains, and the third inoculation is made with a freeze-dried starter culture comprising one or more AAB strains, each subsequent inoculation being carried out, preferably, after a time lag of at least 24 hour of fermentation.

5. The process according to any of claims 1 to 4, wherein said yeast strains are selected from the species *Pichia kudriavzevii,* said LAB stains are selected from the species *Lactiplantibacillus plantarum* and said AAB strains are strains from the species *Acetobacter malorum.*

6. The process according to claim 5, wherein said yeast strain is *Pichia kudriavzevii,* accession number DSM 35384, deposited at DSMZ on March 6, 2025.

7. The process according to claim 5 or 6, wherein said LAB strain is *Lactobacillus plantarum,* accession number DSM 35385, deposited at DSMZ on March 6, 2025.

8. The process according to any of claims 5 to 7, wherein said AAB strain is *Acetobacter malorum,* accession number DSM 35383 deposited at DSMZ on March 6, 2025.

9. The process according to any of claims 1 to 8, wherein, in step d), the inoculated pasteurized cocoa pulp is incubated for 24 hours to 50 hours, preferably for 48 hours.

10. The process according to any of claims 1 to 9 wherein, in step d), the inoculated pasteurized cocoa pulp is incubated at a temperature of between 25°C and 40°C.

11. The process according to any of claims 1 to 10 wherein, in step e), the inoculated pasteurized cocoa pulp is incubated for 24 hours to 50 hours, preferably for 48 hours.

12. The process according to any of claims 1 to 11, wherein, in step e), the inoculated pasteurized cocoa pulp is incubated at a temperature of between 25°C and 40°C.

13. The process according to any of claims 1 to 12 wherein, in step g), said cocoa beans are fermented or unfermented beans.

14. The process according to any of claims 1 to 13 wherein said cocoa beans are dried before performing step g).

15. The process according to any of claims 1 to 14, wherein said fermented pulp is diluted, preferably with water, prior to or during addition to cocoa beans for in field fermentation.

16. The process according to any of claims 1 to 15, wherein said fermented pulp is added to cocoa beans for in-field fermentation at a ratio of fermented pulp (g dry matter) to cocoa beans (g dry matter) of between 1:1 to 1:100.

17. A process of preparing a cocoa product, comprising the steps of (i) fermenting cocoa beans by the process according to any of claims 1 to 16 thereby obtaining a cocoa bean fermentation product, and (ii) using the cocoa bean fermentation product in the preparation of a cocoa product.

18. The process according to claim 17, wherein said cocoa product is selected from the group comprising cocoa nibs, cocoa powder, cocoa extract, cocoa liquor, cocoa mass, cocoa butter and cocoa cake.

19. A process of preparing a chocolate product, comprising the steps of (i) fermenting cocoa beans by the process according to any of claims 1 to 16 thereby obtaining a cocoa bean fermentation product, and (ii) using the cocoa bean fermentation product in the preparation of a chocolate product.

20. The process according to claim 19, wherein the chocolate product is selected from the group comprising chocolate bars, chocolates, truffles, pralines, raw chocolate, stone-ground chocolate, aerated chocolate, chocolate chips, chocolate nuggets, chocolate spreads.

21. A microorganism strain selected from the group consisting of:
*Lactobacillus plantarum,* accession number DSM 35385, deposited at DSMZ on March 6, 2025;
*Acetobacter malorum,* accession number DSM 35383 deposited at DSMZ on March 6, 2025;
*Pichia kudriavzevii,* accession number DSM 35384, deposited at DSMZ on March 6, 2025.

22. A lyophilized starter culture comprising a microorganism selected from the group consisting of:
*Lactobacillus plantarum,* accession number DSM 35385, deposited at DSMZ on March 6, *2025; Acetobacter malorum,* accession number DSM 35383, deposited at DSMZ on March 6, 2025; *Pichia kudriavzevii,* accession number DSM 35384, deposited at DSMZ on March 6, 2025; or any combination thereof.

23. The lyophilized starter culture according to claim 22, further comprising cryoprotecting agents.
